# EUROPEAN PATENT SPECIFICATION

(11) **EP 3 043 761 B1**
(45) Date of publication and mention of the grant of the patent: **21.08.2019**
(21) Application number: 14762036.3
(22) Date of filing: 12.09.2014
(51) Int. Cl.: A61F 13/515, A61F 13/15

(54) **ULTRASONICALLY BONDED ARTICLES AND METHOD OF MANUFACTURING SUCH ARTICLES**
ULTRASCHALL-GEBONDETE ARTIKEL UND VERFAHREN ZUR HERSTELLUNG SOLCHER ARTIKEL
ARTICLES LIÉS PAR ULTRASONS ET LEUR PROCÉDÉ DE FABRICATION

(30) Priority: 12.09.2013 GB 201316225; 23.01.2014 GB 201401179; 07.04.2014 GB 201406208
(43) Date of publication of application: 20.07.2016
(73) Proprietor: Concepts For Success (C4S), 53881 Euskirchen-Stotzheim (DE)
(72) Inventor: SCHMITZ, Christoph, 53881 Euskirchen (DE)
(74) Representative: Plischke, Manfred
(86) International application number: PCT/EP2014/069538
(87) International publication number: WO 2015/036561

(56) References cited:
- WO-A1-2012/042842
- WO-A1-2012/108330
- US-A1- 2005 136 224

## Description

### Field of the invention

The present invention is a disposable article to be worn on the lower torso of a wearer, such as a disposable liquid absorbent article or disposable underwear, as well as a manufacturing method for producing such an article, wherein functional connections, if not all connections are melt-fusion bonds, thusly allowing to produce adhesive free articles.

### Background

In contrast to re-useable garments that are typically manufactured by sewing together textile or fabric materials, manufacturing of disposable article requires combination of various materials at a high production speed, thusly excluding sewing. Presently, such combining is achieved by employing two bonding methods: First, adhesive bonding, i.e. by adding a material that has typically no other function than bonding, such as glues, which are commonly and interchangingly also referred to as adhesives, and secondly melt-fusion bonding, such as by applying thermal (heat embossing), pressure, sonic, especially ultrasonic energy, or a combination thereof.

Addition of glue is generally thought of as a necessary evil, as it adds material cost, complicates production processes, in particular when considering the often-used hot melt adhesives. Further, glues may add undesired smell and even contribute to undesired skin reaction or at least consumer may negatively react to the addition of chemicals. In addition, glues may negatively interact with other elements of the article, such as surfactants or lotions, and may bleed through porous materials, such as nonwovens, or may distort thin films.

Non-adhesive combining of certain components of articles are well known (see e.g. EP1216679, US2009/0133803, or US2011/0004178. It is also known to create simple pants style article without adhesives, such as described in US4745636, wherein a single folded blank is combined with an elastic waist feature to satisfy basic fit requirements. In advanced better fitting articles, such as current baby or adult incontinence products, adhesives are believed to be unavoidable, among others because some of the materials as currently employed are believed to be incompatible with each other for melt-fusion bonding, such as when combining conventional polypropylene and polyethylene, or for the bonding of elastic materials. In WO2002/058612, a method is described wherein a natural fibre core is compressed without inducing any fusion bonding by ultrasonic means and connected to the other components by conventional, i.e. adhesive means. It is also known, to activate adhesives by using ultrasonic energy, such as described in US2005/0136224.

In addition, all melt-fusion bonding methods require positioning the materials that are to be combined between anvils to hold the materials together during or shortly after energy input. This puts particular constraints to the process and equipment arrangement and/or product design.

Recently, new diaper designs have been proposed that provide improved fit without the need for any elastic material, see e.g. EP1865905A1 or WO11/064275 hereinafter referred to as the WO'275 application.

Also recently a new adhesive free melt-fusion bonding method employing a flexible rather than a rigid anvil has been proposed, see EP2621434A1 (C-4-S), hereinafter referred to as the EP'434 application.

The present invention aims at overcoming the shortcomings as resulting from the use of adhesives by combining particular product designs with particular bonding techniques.

In a first aspect, the present invention relates to absorbent articles as a combination of several materials that are functionally bonded by melt-fusion bonds only.

In a second aspect, the present invention relates to a method of manufacturing absorbent articles, in particular body conforming absorbent articles by combining at least key elements of the article only by melt-fusion, preferably ultra-sonic, bonding.

### Summary

In a first aspect, the present invention is a disposable absorbent article to be worn on the lower torso of a wearer as claimed in claim 1. It comprises a crotch region, to be positioned in the crotch region of a wearer during use and a front and a rear waist region extending forwardly and rearwardly to the waist region of a wearer during use. The article comprises a liquid permeable topsheet, a liquid retaining backsheet, and closing element for connecting the front and rear waist regions, further a liquid absorbing core which comprises absorbent, preferably superabsorbent, material, preferably in particulate form, further preferably comprising a first and a second enveloping web, and which is positioned between the topsheet and the backsheet.

In the article, the topsheet, backsheet and absorbent core are functionally connected within themselves and to each other to form an absorbent article by melt-fusion bonds.

The absorbent article according to the present invention may further comprise one or more further web material(s) that is / are functionally connected to the topsheet, backsheet or core by melt-fusion bonds. The further web material(s) is / are selected from the group consisting of
liquid handling webs, preferably selected from the group consisting of
- liquid acquisition materials exhibiting an open pore structure and preferably comprising resilient fibres, more preferably comprising polyester fibres;
- and liquid distribution materials preferably comprising cellulosic or melt-blown fibres;
a further backsheet;
a further topsheet;
side panels;
side panel extensions;
leg hoops;
barrier cuffs.

The absorbent article may further comprise construction bonds for establishing the integrity of the article and optionally application bonds for establishing or maintaining the positioning of the article on a wearer, wherein at least the construction bonds are only melt-fusion bonds.

The article may be an article to be worn on the lower torso of a wearer, preferably being selected from the group consisting of an open diaper, a preclosed and reopenable pants-style article or a closed pants style article, for babies, toddlers or adults, and an article according to the present invention may comprise longitudinal side margins, the longitudinal side margins being overfolded along longitudinal fold lines towards the longitudinally extending centre line of the article and a construction bond for maintaining the overfolding at least in a portion of the crotch region of the article, wherein the construction bond is a melt-fusion bond. It may further comprise a topsheet opening positioned in the crotch region and along the longitudinal centreline, adapted to enable bodily exudates to pass through. Optionally, an article according to the present invention may be inwardly quad folded. Described herein is a method for the manufacturing of a disposable absorbent article or a precursor therefor, whereby the method comprises the steps of
a) providing
   a1)
      - absorbent, preferably superabsorbent material, preferably in particulate form,
      - a first and a second enveloping web;
      - optionally further web material for enhanced liquid handling;
   a2) backsheet web material exhibiting two longitudinally extending side margins, optionally being composed of sub-webs;
   a3i) topsheet web material;
b) preparing individual pieces of absorbent cores by
   b1) positioning the absorbent material and, if present, the further web material for enhanced liquid handling, between the first and the second enveloping web;
   b2) functionally connecting the first and the second enveloping web material;
   b3) separating the connected web material to form individual pieces of absorbent cores;
c) preparing an absorbent article or a precursor therefor by
   c1) positioning the individual pieces of absorbent core between the backsheet and the topsheet material;
   c2) functionally connecting the individual pieces of absorbent core to the backsheet and/or the topsheet;
   c3) functionally connecting the backsheet and the topsheet material while the absorbent core is positioned there between;
   c4) separating the connected backsheet and topsheet material to form individual pieces of absorbent articles or precursor therefor,
whereby the connecting in steps c2), c3), and b2) is executed by applying energy, preferably thermal energy, more preferably sonic energy and most preferably ultrasonic energy to establish melt-fusion bonds which are essentially free of adhesive.

The present invention is also directed towards a method for the manufacturing of an absorbent article to be worn on the lower torso of babies, toddlers or adults, the article being selected from the group consisting of an open diaper, a preclosed and reopenable pants-style article or a closed pants style article. The article exhibits x-, y-, and z-directional extension, longitudinal centre line, front and rear waist regions, each comprising cross-directionally a first and a second region, and a crotch region, and cross-directional centre line in the crotch region.

The method provides individual pieces of an absorbent core manufactured according to steps a1) and b) as described in the above, further comprising the steps of
m) providing
   m1) further backsheet web material, exhibiting a longitudinal centre line which is essentially parallel to the longitudinal centre line of the article and longitudinally extending side margins;
   m2) optionally side panel web materials;
   m3) optionally side panel extension web materials;
   m4) optionally closure tapes
   m5) a backsheet web material;
   m6) a topsheet web material;
n) preparing a chassis sub-assembly from the further backsheet,
   optionally combined with the side panel web materials and/or the side panel extension web materials,
   by applying separation lines to the further backsheet,
   which extend predominantly in the MD and symmetrically to a longitudinally extending centre line of the further backsheet,
   whereby either
   n1) leg hoops are formed by the further backsheet material,
      whilst side panel materials and optionally side panel extension materials are separated from side panel web materials or side panel extension web materials into pieces and added and functionally connected thereto, whereby the separation line
      is positioned at least in a portion of the crotch region less than 9 cm, preferably less than 3 cm, but more than 0 cm away from the longitudinally extending side margins of the further backsheet material
      and is intersecting a further separation line, which extends predominantly cross-directionally from the point of intersection towards but not extending into the side margin of the further backsheet material;
      or
   n2) side panels and leg hoops are formed by the further backsheet material and optionally side panel extension materials are separated from a side panel extension web material into pieces and added and functionally connected to the side panel of the further backsheet
      whereby the separation line is positioned
      at least in a portion of the crotch region less than 9 cm, preferably less than 3 cm, but more than 0 cm away from the longitudinally extending side margins of the further backsheet,
      and being less than 9 cm, preferably less than 3 cm, more preferably less than 1 cm away from and including coinciding with the longitudinal centre line in the front and rear waist region,
      optionally being executed as a perf'n pop separation line;
      or
   n3) side panel extensions, side panels and leg hoops are formed by the further backsheet material, whereby the side panel extensions are separated from the further backsheet material, thereby forming sets of side panel extension pieces
      whereby the separation line is positioned
      at least in a portion of the crotch region less than 9 cm, preferably less than 3 cm, but more than 0 cm away from the longitudinally extending side margins of the further backsheet,
      and being less than 9 cm, preferably less than 3 cm, more preferably less than 1 cm away from and including coinciding with the longitudinal centre line in the front and rear waist region,
      optionally being executed as a perf'n pop separation line.
o) The chassis sub-assembly is combined with the individual pieces of an absorbent core and the backsheet and topsheet, thereby repositioning the sets of side panel extension pieces, when/if present, machine directionally relative to the remaining further backsheet material such that
   o1) the backsheet and topsheet envelope the absorbent core; and
   o2) the further backsheet is positioned on the backsheet on the opposite side of the absorbent core; and
   o3) the optional side panel extensions are positioned between the further backsheet and the backsheet;
p) functionally connecting
   p1) the further backsheet to the backsheet and/or the topsheet;
   p2) if present, the side panel material pieces to the further backsheet and/or the topsheet;
   p3) the side panels as integral parts of the further backsheet to the backsheet and/ or the topsheet in the proximity of the longitudinal side margins;
   p4) if present, the side panel extension pieces to the side panels;
   p5) optionally the absorbent core to the topsheet and/or backsheet;
q) providing a closing element of the article by
   q1) adding and connecting a closure tape for the making of an open diaper or for a pre-closed and reopenable diaper ; or
   q2) positioning the side margins of the front and rear portions of the article in an abutting or overlapping mode for forming a pants style article and connecting the portions;
z) separating a full assembly from the continuous composite so as to form individual articles and optionally further processing such articles.

The functional connecting at least in the steps p1), p2), p3), p5), q), and p4) is executed by applying energy, preferably thermal energy, more preferably sonic energy and most preferably ultrasonic energy to establish melt-fusion bonds.

The process step a3) of providing the topsheet may comprise the sub steps of providing a first and a second strip of topsheet web material, each of the strips exhibiting a cross-directional width of more than half the width of the further backsheet web material, and the process may further comprise the steps of
r1) positioning the first and second strip of topsheet web material such that their laterally outward side margin is essentially in registry with the respective laterally outward margins of the further backsheet and such that the opposite margins of the strips extend beyond the longitudinally extending centreline of the article, such that the strips overlap in the proximity of the longitudinally extending centreline
r2) functionally connecting the strips of topsheet material with the further backsheet material in the proximity of and along the longitudinally extending side margins;
r3) overfolding the opposite side margin of the strip of topsheet material outwardly over and beyond the connecting of the strip of topsheet material to the further backsheet;
r4) functionally connecting the overfolded portions of the strips of topsheet material to the underlying portion that is already connected to the further backsheet;
r5) folding the resulting subassembly along the longitudinal centreline such that the overfolded portions of the first and the second strip are positioned towards each other and the portions of the strips extend beyond the further backsheet;
r6) functionally connecting the portions of the first and the second strip, that extend beyond the connecting to the further backsheet, to each other;
wherein the functional connecting in steps r2), r4), and r6) is executed by applying energy, preferably thermal energy, more preferably sonic energy and most preferably ultrasonic energy to establish melt-fusion bonds.

The step r6) may be executed such that the functional connecting of the portions of the first and the second strip is executed intermittently such that upon donning of the article on a wearer openings between the first and the second strip are created, which can be positioned in registry of body openings to allow bodily exudates to pass through The method for manufacturing a disposable absorbent article or a precursor therefor may further comprising the steps of applying at least one further web material selected from the group consisting of
t1) liquid handling webs, preferably selected from the group consisting of
   - liquid acquisition materials exhibiting an open pore structure and preferably comprising resilient fibres, more preferably comprising polyester fibres;
   - liquid distribution materials preferably comprising cellulosic or melt-blown fibres;
t2) a further topsheet;
t3) side panel extensions;
t4) leg hoops;
t5) barrier cuffs,
wherein the further web materials are functionally connected by applying energy, preferably thermal energy, more preferably sonic energy and most preferably ultrasonic energy to establish melt-fusion bonds.

All connecting is executed without using glues.

### Brief description of the Figures

Fig. 1 shows schematically an absorbent pad as an absorbent article or as a precursor for such an article according to the present invention.
Fig. 2A to 2P show schematically chassis elements and how such chassis elements can be combined according to the present invention.
Fig. 3 shows schematically an execution of an absorbent article in a pants-style shape according to the present invention including a topsheet opening and overfolded side margins.
Fig. 4 A-P depict schematically stages of combined elements for forming articles according to the present invention.
Fig. 5 A to H depict schematically process steps for forming an inwardly quad folded article, such as a recloseable diaper.
Fig 6. depicts schematically a conventionally quad folded article.
Fig. 7 depicts schematically various arrangements for a preclosed and reopenable pants-style article.
Fig. 8 and Fig. 9 schematically depict the process steps for making an article according to the present invention.
Fig. 10 depicts schematically connecting modes for web materials.

In all figures, same numerals denote identical or corresponding features.

### Detailed description

"Articles to be worn on the lower torso of a wearer" refers to articles such as pants, panties, or diapers and the like, which do not require other support means for being held in a position between the legs of a wearer, and which extend towards the waist region of the wearer and encircle legs or hips or waist of the wearer. The articles comprise a crotch region to be positioned in the crotch region of a wearer during its intended use, as well as a front and a rear waist region extending forwardly and rearwardly to the waist region of the wearer during use. Corresponding left and right front and rear waist regions are connected during use by side panels forming a waist hoop. An article may have rear or front side panels only, or both front and rear side panels. Further, the article may comprise side panel extensions, as may be positioned between left and right side panels. An article may be an "open style" article, such as well known from disposable diapers, where front and rear side panels are - optionally releasably - connected to each other such as by adhesive, preferably mechanical fastening means. An article may be a pants style article, where all elements forming the waist hoop are essentially permanently connected, i.e. the connection can essentially only destructively opened, such by tearing it open. Articles can also be of the preclosed diaper type, where a pants style article is manufactured with releasable connections in the waist hoop, such that a user may be able to open and reclose the connection.

"Three dimensionally (3D) shaped articles" refers in the present context, which are conforming to the body contours of a wearer by employing a "darting" effect, such as well-known in sewing of garments, and as described in more detail e.g. WO2011/064275, to which express reference is made in this context.

"Elastic free 3D shaped articles" refers to articles that do not require elastic elements to achieve a 3D shape.

"3D shaped articles with extensible materials" refers to articles comprising materials that are extensible so as to conform to certain body contours without exerting significant pressure.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training or absorbent pants, diaper holders and liners, sanitary napkins and the like. The present invention also relates to precursors as may be used to manufacture articles that comprise further elements, as may use or not use the present invention.

"Diaper" refers to an absorbent article generally worn by infants and adult persons that suffer from urine or faecal incontinence or by females during menstruation period about the lower torso comprising closure elements that allow to position the article on a wearer, typically referred to as closure or fastening tapes or means, such that these articles take a pants-like shape. Optionally such diapers may also be delivered in a closed, but undestructively reopenable configuration and may be referred to as reopenable pants. In contrast, pants that are closed articles that can be opened such as for removal only by destructively tearing e.g. of the side seams, are referred to as tear-open pants.

"Disposable" is used herein to describe articles that are generally not intended to be laundered or otherwise restored, i.e., they are intended to be discarded after use and, preferably, to be recycled, composted or otherwise disposed of in an environmentally compatible manner. It should be noted, that disposable articles may be used several times, such as wearing disposable underwear over several days, or re-using disposable absorbent articles if these have not been loaded at the first use occasion. It should also be noted, that disposable articles may be washable, and may actually be washed for a limited number of wash-cycles, typically not more than ten wash cycles.

"Comprise," "comprising," and "comprises" is an open-ended term that specifies the presence of what follows, e.g. a component, but does not preclude the presence of other features, elements, steps or components known in the art, or disclosed herein.

The term "web material" refers to an essentially endless material in one direction, i.e. the longitudinal extension, or the length, or the x-direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Often, though not necessarily, the width of web materials (the y-direction) will be significantly larger than the thickness, but less than the length. Often, though not necessarily, the thickness and the width of such materials is essentially constant along the length of the web. Such web materials may be without intending any limitation, cellulosic fibre materials, tissues, films, woven or non-woven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, non-woven/film laminates. A web material may further be composed of sub-webs, such as when two sub-webs are provided in parallel, optionally upon longitudinal separation form a unitary web. Such sub-webs may be connected to each other, such as along their longitudinal side margins. Web materials may comprise other materials, such as added particles, hydrophilizing agents and the like.

The terms "superabsorbent", "super absorbent material" or "SAM", sometimes also referred to as "absorbent gelling material" or "AGM", "absorbent polymer material" are used herein interchangingly, and refer to partially cross-linked polymeric materials, which can absorb water whilst they are swelling to form a gel. Typically, though not necessarily, such materials are delivered in a particulate form. Alternatively, superabsorbent materials may be delivered in another form, such as in a fibrous form. The present invention is particularly suitable for being used in continuous production processes, and even more so when such processes are operated at high production speed, that may exceed 500 m / min.

The structures according to the present invention are particularly useful for disposable absorbent articles, such as, but not limited to, disposable baby diapers, training pants, adult incontinence article, feminine hygiene articles and the like. Such articles have very varying requirements as to the desired absorbency, depending on the intended use and/or user. For disposable underwear articles, the absorbency may be negligible.

Various elements of the articles according to the present invention are connected to each other, such as when an article comprises a backsheet and a topsheet. Within the context of the present invention, connections that establish the integrity of the article or its precursor during manufacturing, before and during use are referred to as construction bonds. In addition thereto, an article may comprise other bonds to establish or to maintain the positioning of the article on a wearer, which may be referred to as application bonds, and which may be mechanical faster or adhesive tapes in the case of openable diapers or adhesive layers applied to the garment oriented surface of the article in the case of pads for being inserted into underwear. Also, the present invention may provide articles of the reopenable pants style, wherein preferably the side panels are closed during manufacturing, preferably comprising recloseable fastening means so as to allow opening and closing by a user.

It is a key feature of the present invention to provide alternative bonding to conventional adhesive or glue bonding at least for construction bonds by employing melt-fusion bonds. The term melt-fusion bond or bonding refers to the connecting of two or more strata or layers that may belong to one and the same material, such as when a web is overfolded, or to different materials, such as when two or more layers of materials are positioned onto each other. By addition of energy, at least one of the materials starts melting or at least becomes plastically deformable. Representative materials are generally referred to and well known to a person skilled in the art as thermoplastic materials, such as without intending any limitation polypropylene or polyethylene. Upon application of pressure, the molten or plastically deformable material forms a consolidation region, which can be considered to belong to both materials or layers. However, not necessarily all materials that are to be connected need to form melt-fusion bonds with each other, but melt-fusion bondable materials may be combined with materials that exhibit a higher melting temperature or are essentially non-plastically deformable.

There are various and non-exclusive ways to apply the energy to the materials, such as compressing between rolls, one of which or both may be heated and thusly induce the temperature increase. Optionally the materials may be heated prior to be in contact with the rolls, or the rolls may apply sufficient pressure so as to increase the temperature up to the plastic deformation or even melting temperature.

A preferred method is by using the well-known sonic or ultra-sonic bonding, where the materials are run through a gap between an ultrasonic horn and an anvil and energy is primarily or exclusively applied to the web via vibration of the horn.

A particularly preferred execution is described in the EP'434 application to which express reference is made. Therein, the anvil is executed as a flexible anvil, as may be realized by using helical anvil elements, such as springs, which may be positioned around the circumference of a cylindrical roll acting as anvil carrier. Without wishing to be bound by a theory, it is believed that the flexibility of the anvil elements and further the shape of the helix, if used, create very strong yet soft consolidation regions. Such a technique also enables to connect through materials that are non-meltable, such as paper, or materials of high plastification or melting temperatures, such as polyester webs, to melt-fusionable webs, such as polypropylene webs.

Thus, such melt-fusion bonding can replace conventional adhesive or glue bonding. The term adhesive or glue bonding refers to the connecting of two or more strata that may belong to one and the same material, such as when such a web is overfolded, or to different materials, such as when two or more layers of materials are positioned onto each other by adding a third material, the adhesive or glue, between the surfaces. Such an adhesive may be instantaneously tacky or may become tacky upon activation such as through energy or pressure, or may lose its tackiness upon cooling or ageing.

It is a principle of adhesive bonding that forces are transmitted from one material to the other via the adhesive - consequently an adhesive bond may fail "adhesively", i.e. the bond between the adhesive material and one of the connected materials fails, or "cohesively", i.e. the failure occurs within the adhesive. In contrast, melt-fusion bonds do not require any further material in the consolidation region and the failure mechanism is either breakage of the consolidation region, in the transition region from the consolidated material to the surrounding material, or the bonding may actually increase the local strength and the material failure occurs in the surrounding of the bond. Further, an adhesive connection only bonds two facing surfaces of two materials to each other, even if, for example an adhesive applied to both surfaces of a web and two other webs are bonded thereto, or even if a web is completely saturated with adhesive to then connect on both surfaces to webs, as also in such cases the bond properties are determined by the properties of the two adjacent surfaces and the adhesive. In contrast, a melt-fusion bond such as may be created by ultrasonic bonding connects through the complete material - and may even connect through more than two material layers.

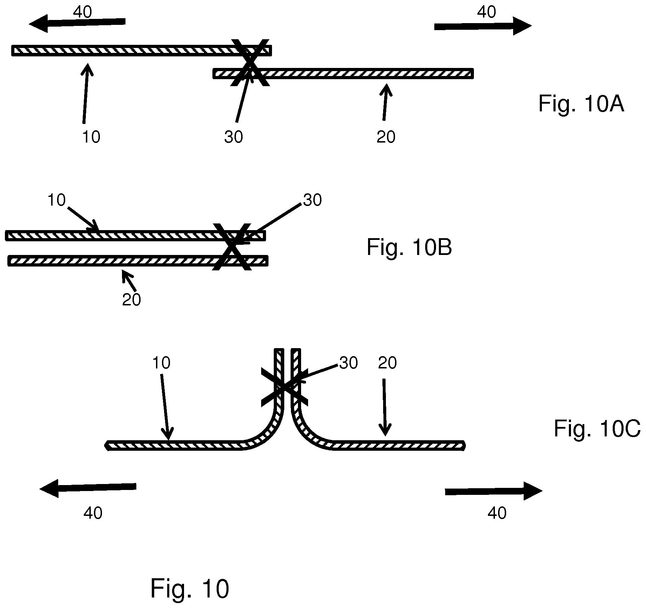

Bonding two or more webs or strata of a folded web can be made in an "overlap" or in an "abutting" mode. When referring to Fig. 10, a first 10 and a second 20 web can be positioned such that their side margins 11 resp. 21 that are in the proximity of the connecting bond point 30, are on the opposite sides of the bond point, forming a so called "overlap bond", see Fig. 10A, or the same side of the bond point, forming a so called "butt connection", see Fig. 10B. When pull forces are applied to the webs, such as indicated by pull force arrows 40 in Fig. 10 of the butt mode execution, the skilled person will realize that the overlap mode creates mostly shear forces in the connecting point, but peel forces in the butt mode. Adhesive connections are much more prone to failure under peel forces as compared to shear forces, i.e. an overlap mode connection will be stronger than a butt mode connection. For melt fusion bonds, however, this difference in bond strength is much less pronounced.

Within the present context, the term "functionally connected" refers to the strongest connection. Thus, in the case of having both melt-fusion bonds and adhesive bonds, the melt-fusion bond is considered the functional connection, if it fails under an increasing load after the adhesive bond fails. For example, adding a small amount of a glue or low failure force glue that fails before the melt-fusion bond fails or the material tears leaves the melt-fusion bond as the "functional connection". Thus, functionally connected refers to the fact, that even if additional bonding by adhesives or glues would be applied that would not improve the function of the article, i.e. the article would maintain its integrity and main functionality even without these glues or adhesives.

Thus in the extreme, the present invention relates to an article that is essentially free of adhesive or glue, at least for the functional construction bonds.

Articles according to the present invention comprise at least a first web material, as may be referred to as backsheet, as may be positioned such that it is oriented away from the wearer during use, a second web material, as may be referred to as topsheet as being oriented towards the wearer during use. Side panels can be added as a separate material, which is connected at least to the backsheet in the front and/or rear regions of the article along the longitudinally extending side margins of the backsheet whilst extending cross-directionally inwardly, i.e. from the longitudinally extending side margins towards the longitudinally extending centre line. Alternatively, side panels may be cut out of the backsheet material, then referred to as "integral" side panels, such that a strip of backsheet material in the proximity of the longitudinally extending side margins remains, but a window-like part is cut out towards the longitudinally extending centre line, without going beyond this line. Already during the manufacturing process or by the end user upon donning, the separate or integral side panels, optionally with side panel extensions, are folded outwardly along a predominantly longitudinally extending fold line to create the waist hoop. This outward folding turns portions of the backsheet that were oriented towards the wearer into their opposite orientation, i.e. away from the wearer, or vice versa. The articles may further comprise side panel extensions, as may be functionally connected to the outwardly positioned edge of the side panel in an in-use position, and serve as a length extension of the side panel so as to allow for a wider waist circumference. Such side panel extensions may be added as separate web materials, optionally exhibiting different properties such as elasticity, and may be applied in a single layer or in a folded or even "leporello" type arrangement. The may also be added as a cut out material from other webs during manufacturing, as will be discussed in more detail herein below.

As articles according to the present invention are intended to be worn on the lower torso of a wearer without the need for additional fixation means, such as when an absorbent pad is inserted into and maintained in position by underwear, the article comprises closing elements. In case of an open type diaper, such elements may be conventional closure tapes, preferably of the well-known hook and loop type, and more preferably manufactured without adhesive lamination but melt fusion bonding. The hook materials may engage with fibrous, e.g. nonwoven materials of the article, such as textile backsheets, or with specially applied landing zones, as may be applied to the other web materials prior to the combination as discussed herein in more detail. Also the hook materials may be applied as fastening tapes to the side panels or side panel extensions, as required at various point of the manufacturing process. Optionally the mechanical fasteners may be already connected during the manufacturing so as to form a closed, but re-openable pants style article. A further closing element is a side seam, as well known in the art, connecting front and rear portions of the side panels or side panel extensions, respectively so as to form closed pants style article. In this case, the opening may be done destructively, such as by tearing the side seam open. The connecting of any of the closing elements is preferably executed by melt-fusion bonds.

In particular for pre-closed or side seamed pants style articles, any of the backsheet material, side panel material, side panel extension material, or fastening tape may be executed as partially or fully elasticated materials, as well known in the art..

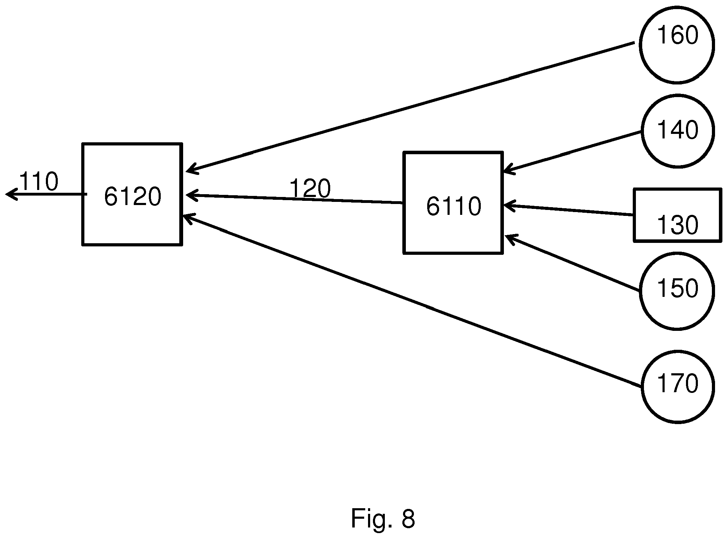

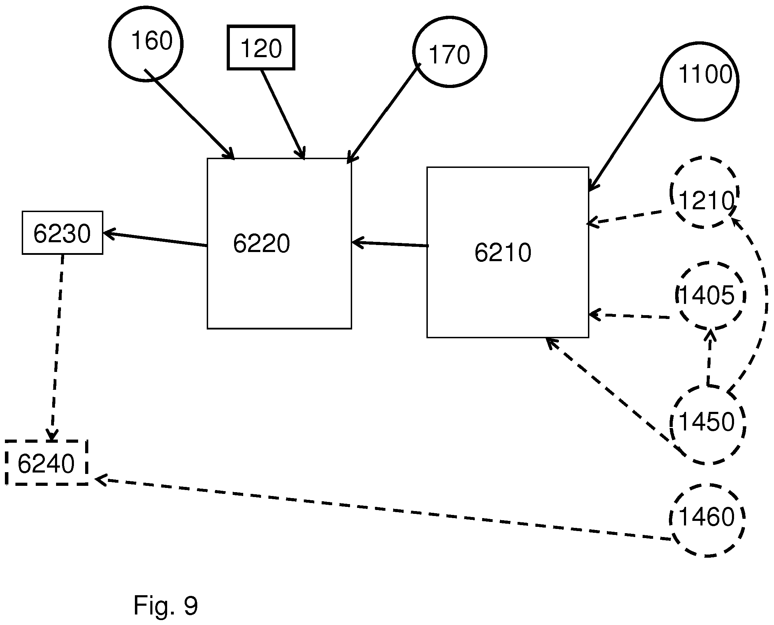

The manufacturing of absorbent article according to the present invention is now further explained by referring to Fig. 8 and 9. The articles, which may be an open diaper, a preclosed and reopenable pants-style article or a closed pants style article, exhibit an x-, y-, and z-directional extension, longitudinal centre line, front and rear waist regions, each comprising cross-directionally a first and a second region, and a crotch region, and a cross-directional centre line in the crotch region.

For the forming of an absorbent core 120, absorbent material 130 is combined with enveloping web materials 140, 150, such as in a core forming unit 6110 preferably by the process as described in more detail in the above reference patent application WO2014/001487 or WO2014/001488. Additional materials may be added as well known to a skilled person, such as without limitation materials to enhance liquid handling, odour absorption, and the like. The enveloping materials and optionally also other materials are functionally connected, preferably by applying energy, preferably thermal energy, more preferably sonic energy and most preferably ultrasonic energy to form melt fusion bonding. In the absorbent pad combining unit 6120, discrete pieces of the absorbent cores 120 are preferably spaced apart and combined with a conventional topsheet 160 and conventional backsheet 170 to form an absorbent pad 110, as may be used as such, such as for an insert, or may form a precursor for an absorbent article, such as when being combined with further elements. The connecting to hold the elements of the article together is also preferably executed by applying energy, preferably thermal energy, more preferably sonic energy and most preferably ultrasonic energy to form melt fusion bonding.

Referring now to Fig. 9, an article that may be worn like a pants style article may be manufactured by providing the appropriate materials, namely an absorbent core 120, preferably formed by the process described in the above, conventional topsheet 160 of the nonwoven or apertured film type and conventional liquid impermeable backsheet 170, as may be a film and as may be at least partially elastic, a particularly treated nonwoven, or a composite thereof. Backsheet 170 may be provided as a unitary web, or may be composed of sub-webs, such as two webs of essentially half the width. The process may further include the providing of a side panel web material 1210, a side panel extension web material 1405, closure tapes 1450 and other conventional elements of an absorbent article well known to a person skilled in the art, such as a further topsheet, further leg hoops or leg cuff barriers etc. (not shown).

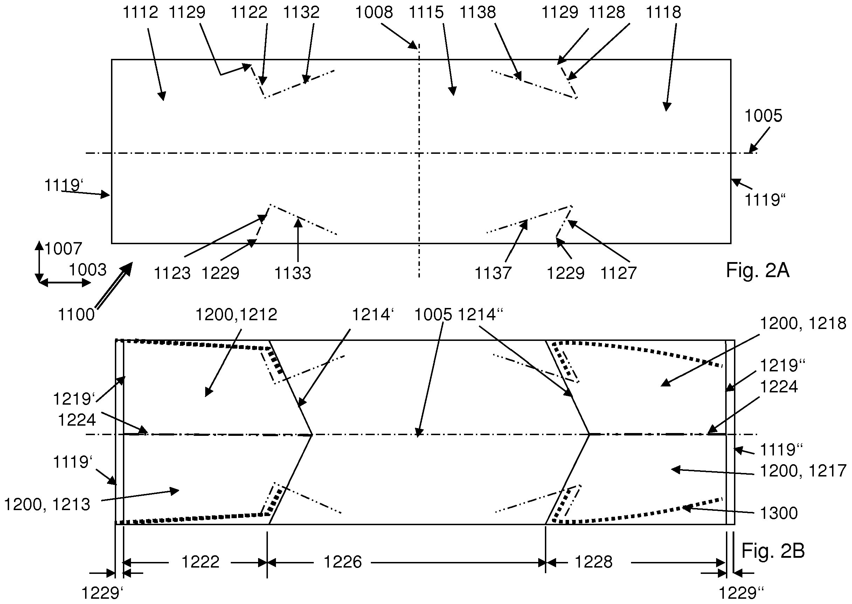

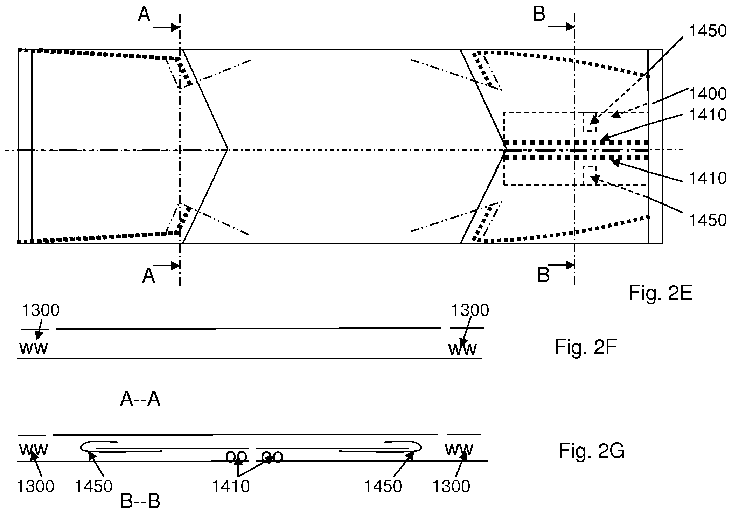

The further backsheet 1100 is prepared in a step 6210 by applying separation lines to a further backsheet web material 1110 which extend predominantly in the MD and symmetrically to a longitudinally extending centre line of the further backsheet, as described in the above. The step can be executed as one of three alternatives:
In a first one, corresponding to products as depicted in Fig. 2 A, B, E, and F, leg hoops are formed by the further backsheet material(s) 1100, whilst pieces of side panel materials and optionally side panel extension materials are separated from side panel web materials 1120 or side panel extension web materials 1405 and added and functionally connected to the further backsheet. The separation line is being applied such that it is positioned at least in a portion of the crotch region less than 9 cm, preferably less than 3 cm but more than 0 cm away from the longitudinally extending side margins of the further backsheet material, and such that it intersecting a further separation line, which extends predominantly cross-directionally from the point of intersection towards but not extending into the side margin of the further backsheet material.

In the second alternative, side panels and leg hoops are formed by the further backsheet material 1100 and optionally side panel extension pieces 1400 are separated from a side panel extension web material 1405 and added and functionally connected to the side panels 1200, which are integral elements of the further backsheet 1100. The separation line is positioned at least in a portion of the crotch region less than 9 cm, preferably less than 3 cm, but more than 0 cm away from the longitudinally extending side margins of the further backsheet, and being less than 9 cm, preferably less than 3 cm, more preferably less than 1 cm away from and including coinciding with the longitudinal centre line 1005 in the front 1112 and rear 1128 waist region. Optionally, this may be executed as a perf'n pop separation line, i.e. an intermittent separation line with some material remaining unseparated, so as to provide a certain strength, which is reduced compared to the surrounding regions such that e.g. upon applying forces as applied during the process or by a user, the separation becomes complete.

For the third alternative, side panel extensions, side panels and leg hoops are formed integrally by the further backsheet material 1100, whereby the side panel extensions are separated, such as cut out, from the further backsheet material 1100, thereby forming sets of side panel extension pieces 1412, 1413, 1417, 1418. In case of the further backsheet material being provided as a unitary web, the separation line is positioned at least in a portion of the crotch region less than 9 cm, preferably less than 3 cm, but more than 0 cm away from the longitudinally extending side margins of the further backsheet, and being less than 9 cm, preferably less than 3 cm, more preferably less than 1 cm away from and including coinciding with the longitudinal centre line in the front and rear waist region. In case of providing the further backsheet material as two sub-webs of half the width, the separation line is positioned at least in a portion of the crotch region less than 9 cm, preferably less than 3 cm, but more than 0 cm away from the longitudinally extending side margins of the further backsheet, and being less than 9 cm, preferably less than 3 cm, more preferably less than 1 cm away from and including going beyond the longitudinal centre line in the front and rear waist region. Optionally, this may be executed as a perf n pop separation line, i.e. an intermittent separation line with some material remaining unseparated, so as to provide a certain strength, which is reduced compared to the surrounding regions such that e.g. upon applying forces as applied during the process or by a user, the separation becomes complete.

The chassis sub-assembly is combined in a step 6220 with pieces of absorbent core 120, topsheet 160 and backsheet 170.

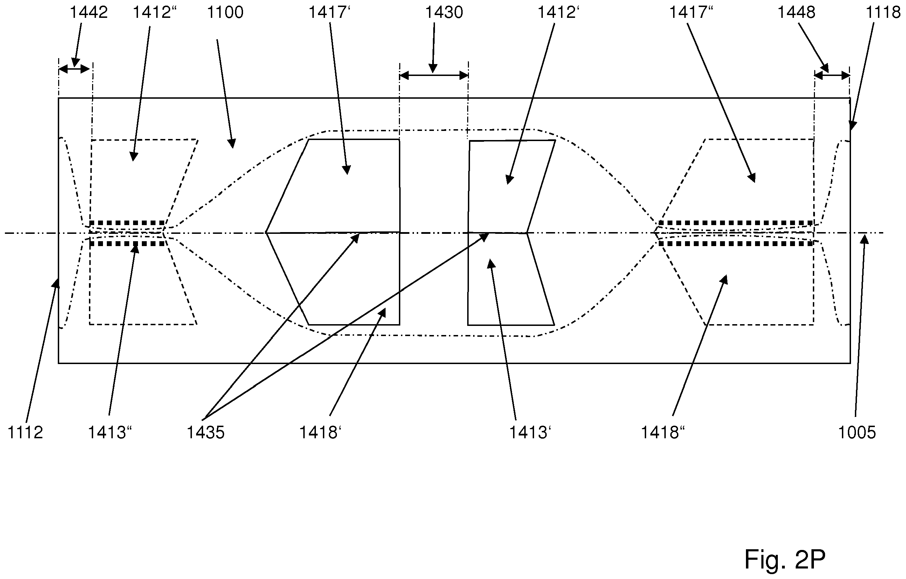

If the side panel extension pieces 1412, 1413, 1417, 1418 are separated from the further backsheet, these are repositioned preferably by a process as described in more detail in WO2016/038170, to which express reference is made. To this end, the separation lines are applied to the further backsheet as depicted in Fig. 2P with dash-dotted lines, such that the further backsheet web material 1110 remains essentially continuous and the side panel extension pieces are separated each around its full perimeter. This may be executed on a vacuum supported anvil drum, against which a die cutting unit operates and performs the separation. As shown in the execution of Fig. 2P, there are four side panel extension pieces of arbitrary shape, limited by the space requirements for leg hoops and side panel materials. In the figure, the pieces are shown in solid lines in the position prior to repositioning, namely first 1417' and second 1418', e.g. left and right, rear side panel extensions and first 1412' and second 1413' front side panel extension pieces, and with dashed lines in the position after repositioning, and denoted 1417", 1418", 1412" and 1413", respectively, whereby prior to the repositioning the regions of the front side panel extension pieces are positioned rearwardly of the regions of the rear side panel extension pieces. Front and rear side panel extension pieces are separated by side panel extension MD-spacing 1430, which corresponds to the sum of the front side panel extension margin length 1442 and the rear side panel extension margin length 1448. First and second side panel extension pieces are separated by a separation line 1435 essentially coinciding with the longitudinal centre line 1005, or by separate predominantly longitudinally extending separation lines (not shown). It should be noted that the side panel extension pieces may have various shapes.

Whilst the vacuum holds the side panel extension pieces on the anvil drum, the remaining continuous part or parts of the further backsheet material web 1110 is /are withdrawn from this drum towards guide rolls and back to the anvil drum, thereby creating a machine directional offset such that the rear side panel extension pieces are placed on the rear portion of same further backsheet, whilst the front side panel extension are placed on the front portion of the subsequent further backsheet. Optionally, the side panel pieces may be additionally transferred to a cooperating combination drum, where they are married to the remaining continuous further backsheet web material as described.

The arrangement of the various elements in the article after the article assembly step 6230 is such that
1) the backsheet 170 and topsheet 160 envelope the absorbent core 120; and
2) the further backsheet 1100 is positioned on the backsheet 170 on the opposite side of the absorbent core 120; and
3) the optional side panel extensions 1400 as may be separated from a side panel extension web material 1405 are positioned between the further backsheet 1100 and the backsheet 170.

The article comprises the following functional connections, which may be applied prior to or during the article assembly step 6230, or in a separate assembly connecting step 6140, i.e. some of these connections may have been executed already earlier in the process flow, and some or all during or after the article assembly step 6230:
1) the further backsheet 1100 to the backsheet 170 and/or the topsheet 160;
2) if present, the side panel material pieces 1400 to the further backsheet 1100 and/or the topsheet 160 or backsheet 170;
3) the side panels 1200 as integral parts of the further backsheet 1100 to the backsheet 170 and/ or the topsheet 160 in the proximity of the longitudinal side margins;
4) if present, the side panel extension pieces 1400 to the side panels 1200;
5) optionally the absorbent core 120 to the topsheet 160 and/or backsheet 170.

The article is further equipped with one or more closing elements, which may comprise the steps of adding and connecting a closure tape for the making of an open diaper or for a pre-closed and reopenable diaper or the positioning the side margins of the front and rear portions of the article in an abutting or overlapping mode for forming a pants style article and connecting the portions, such as in side panel closing step 6240.

It is an important element of the present invention that the functional connecting at least of the connections 1), 2), 3) and 4) and preferably also for any other connecting is executed by applying energy, preferably thermal energy, more preferably sonic energy and most preferably ultrasonic energy to establish melt-fusion bonds.

The principles of articles according to the present invention are now explained by referring to particular executions. These should, however, not be seen limiting in anyway and elements described in one of the executions may be combined with elements of other executions or used on their own.

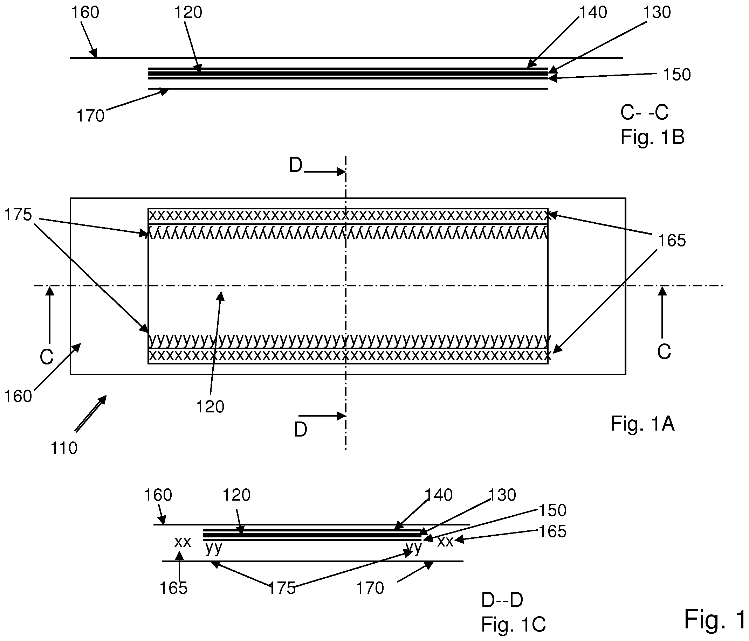

In a first non-limiting embodiment as depicted in Fig. 1A, with cross-sections depicted in Fig. 1B and C, an absorbent article is an absorbent pad 110, such as may be placed as such in the underwear of a wearer or the absorbent pad may be used as a precursor for an absorbent article, as described herein below.

Thus the absorbent pad 110 comprises an absorbent core 120 comprising absorbent material 130 as being enveloped between a first 140 and a second envelop 150 web, essentially coextending with the absorbent material 130 or - such as in case the absorbent material consists essentially of particulate material - with the deposition region of this material. Optionally and often preferably, the absorbent material may be in particulate form, and at least a portion thereof is placed into pores of a porous, often fibrous material, which may also have absorbent and/or fluid handling properties, and both the absorbent material and the porous material are enveloped. Optionally and often preferably, further materials, such as liquid handing materials may be included in the absorbent core or the absorbent pad, preferably selected from the group consisting of liquid acquisition materials exhibiting an open pore structure and preferably comprising resilient fibres, more preferably comprising polyester fibres and liquid distribution materials preferably comprising cellulosic or melt-blown fibres.

Preferably, such an absorbent core as well as suitable ways to manufacture such a core are employed that are described in co-pending applications WO2014/001487 (C-4-S; Schmitz) and WO2014/00148 (C-4-S; Schmitz), to which express reference is made, wherein such cores are made by functional melt-fusion bonding without the need to add any adhesive or glue but yet allowing pre- and in-use immobilization of the material without negatively impeding the liquid absorbency.

Such a core may then be positioned between a preferably liquid impermeable backsheet 170 and a preferably liquid permeable topsheet 160, that at least in length direction extend over the absorbent core, and can be functionally melt fusion bonded to these, here indicated by connecting lines 175 (indicated with symbols "yy") between the absorbent core 120 and the backsheet 170 and connecting lines 165 (indicated with symbols "xx") between backsheet 170 and topsheet 160.

Thus, the subassembly exhibits good integrity, i.e. it does not fall apart, without the use of any adhesive for achieving functional bonds, and - as melt-fusion bonds are inherently insensitive to moisture - even when the subassembly is wetted.

Such a subassembly can essentially form an absorbent article, such as an absorbent pad, optionally equipped with conventional add-ons, such as wings to be folded around the underwear of a wearer, or application means to establish or maintain the article in the appropriate position during use, such as mechanical fastener connections that allow melt-fusion bonding and thus a completely glue free article, or an application glue on the garment oriented surface.

Such a subassembly may also be a precursor for further converting into an absorbent article, such as an openable diaper or a recloseable pants style diaper.

To this end, further elements are added to the absorbent pad, such as side panels or ears that allow affixing the product around the waist of a wearer. A particular preferred execution includes leg hoop features that improve the fit of the article around the legs of a wearer. Such leg hoop features may result from adding separate leg hoop materials, or such leg hoops may be created integrally from other article layers, such as by applying separation lines to a backsheet material.

In this regard, express reference is made to the above referenced EP1865905A1, describing the function of a leg hoop, which is positioned around the upper thigh of a wearer. Such designs allow body conforming fit without conventional longitudinally extending leg elastics, resulting in various benefits for the manufacturer and end-user.

In the present context, such a leg hoop functionality can readily be implemented by employing melt-fusion bonding only.

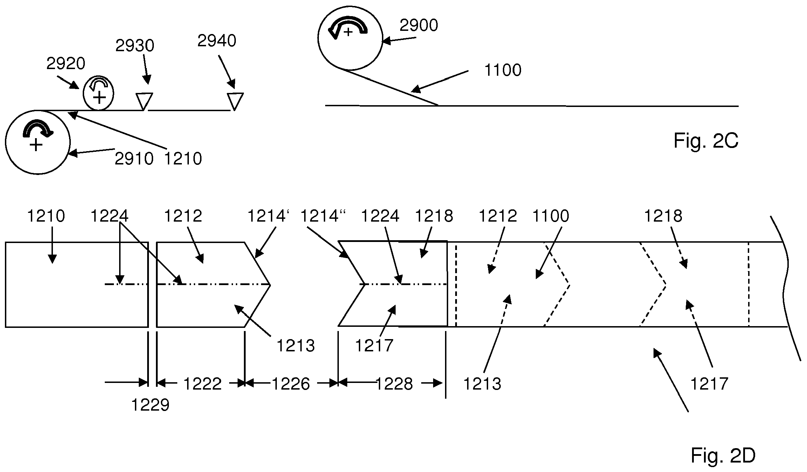

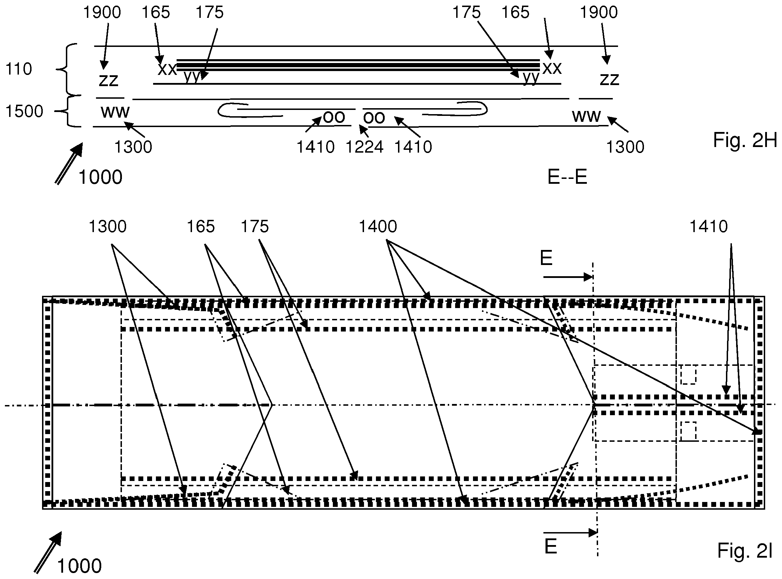

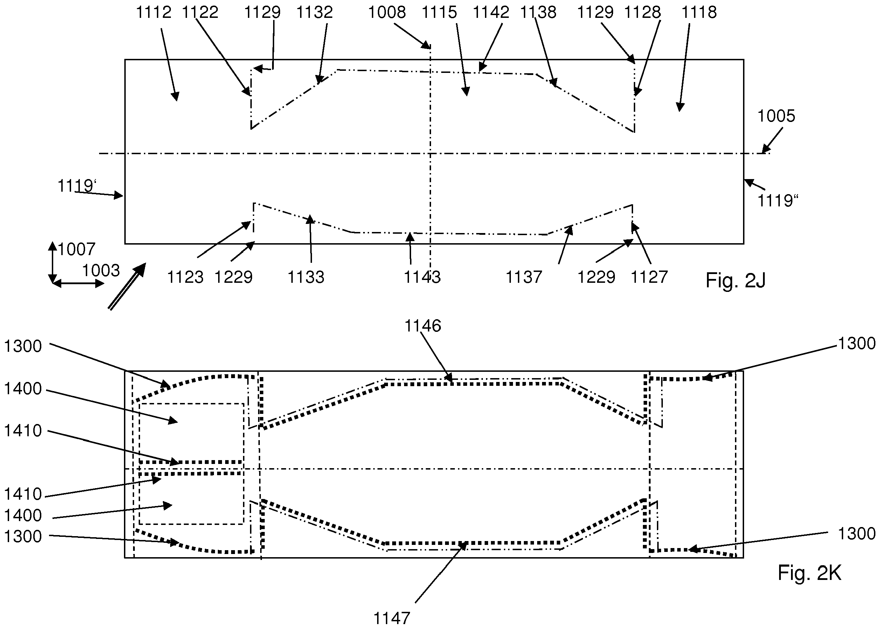

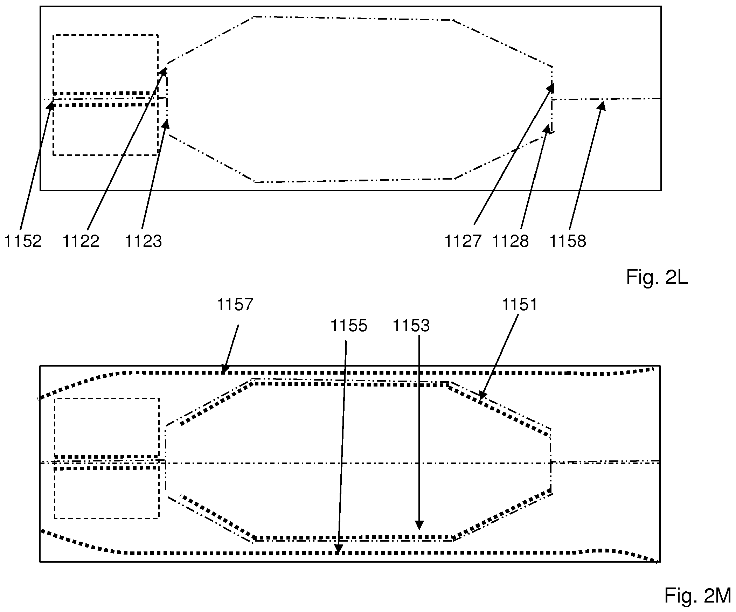

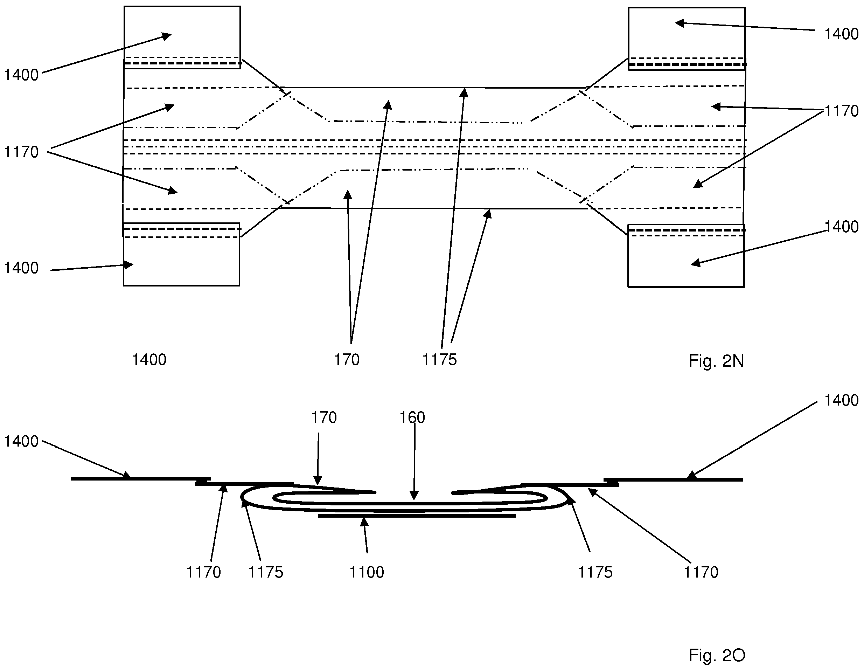

Referring to Fig. 2, a further backsheet 1100 of an article (1000 in Fig. 2I) is shown in Fig. 2A with a longitudinal direction 1003 and longitudinal centre line 1005 as well as a corresponding cross-direction 1007 for both the article and its elements. As the resulting article, the further backsheet has a front 1112 and a rear region 1118 to be positioned towards the front and rear waist of a wearer during its intended use as well as a crotch region 1115 to be positioned in the crotch region of a wearer during use, wherein a cross-directional centre line 1008 is typically though not necessarily positioned exactly in the middle of the article, such as for allowing an asymmetric fit, e.g. fitting higher in the back waist region of a wearer than in the front waist region. The further backsheet may be separated from a backsheet web material along a cross-directional separation line 1119, which will create the front 1119' and rear 1119" end margins of the further backsheet.

Fig. 2A further shows four first separation lines 1128, 1127, 1122, 1123 that extend from the longitudinally extending side margins or preferably from the proximity thereof towards the longitudinal centre line but without intersecting it, i.e. predominantly in cross-direction and preferably leaving an unseparated stripe 1129 at the longitudinally extending margin, that may have a width of more than 1 mm and less than 40 mm.

Within the present context, the term "predominantly extending in one direction" means that a line, a cut, or a separation line has a projection onto the longitudinal or cross-direction that is larger than its projection onto the direction perpendicular thereto. Within the present context, the term "proximity" means that something is close to something else, generally at a distance of more than 1mm, but less than 40 mm.

Fig. 2A also shows four second separation lines 1138, 1137, 1132, 1133, starting at the intersection with the first separation lines, and extending predominantly longitudinally towards the cross-directional centre line, but not intersecting it. Depending on the specific design of the article, these second separation lines may have a length as projection to the cross-direction of from at least 5 mm, preferably more than 15 mm and often more than 20 mm, but not more than about 50 mm

Alternatively, the first and the second separation lines may be replaced by a single separation line, such as a rounded cut, with a first part pointing towards the longitudinal centreline, and a second part pointing towards the cross directional centreline can be implemented. The material between the first and the second separation lines on each longitudinally extending margin can - upon appropriate connecting to the side panels - function as leg hoop during use.

Yet a further option for execution the separation lines is depicted in Fig. 2J, with third separation lines 1142 and 1143 essentially connecting the front and rear second separation lines 1132, 1133, resp. 1138, 1139 by third separation lines 1142 resp. 1143. These lines run along and in the proximity of the longitudinally extending side margins in the crotch region 1115. Preferably, though not necessarily they are straight lines. Fig. 2J further shows the first separation lines 1128, 1127, 1122, 1123 to be cross-directionally oriented, as may allow easy processing.

Also in the design as depicted in Fig. 2J, the first, second and third separation lines may be formed independently from each other or concurrently, optionally by the same separation means such as knife / anvil system, ultrasonic separation, laser cutting or the like. Alternatively, to the executions as discussed in the context of Fig. 2A to J, in a further execution as explained in the context of Fig. 2L to 2M, the front and or rear side panels are made as a part of the further backsheet 1100 rather than a separately provided web material. To this end, the further backsheet may be cut by a cutting pattern as depicted in Fig. 2L and 2M, wherein the first separation lines 1128, 1127, 1122, 1123 are running from the endpoints of the second separation lines towards the longitudinally extending centre line, there the front and rear ones intersect, respectively. A front 1152 and rear 1158 fourth separation line separates the further backsheet in the regions which correspond to the side panel regions, e.g. in Fig. 2L. Alternatively, the front and/or rear separation lines may be formed by two essentially parallel separation lines, indicated for the rear part only by lines 1158' and 1158", such that a strip of material remains along the longitudinally extending centre line. The connecting lines 1151 and 1153 are positioned laterally inwardly and in the proximity of the second and third separation lines on either side of the centre line. Further connecting lines 1155 and 1157 now run essentially continuously from the front to the rear, in the crotch region between the longitudinally extending side margin and the third separation lines. Preferably, these further connecting lines are curved in the front and/or rear regions.

As will be appreciated, the further backsheet comprises a section that will be doubling the backsheet material in the article, refer to the further backsheet crotch centre piece 1190 in Fig. 2L. In yet a further execution of the present invention as explained by exemplarily referring to Fig. 2P, this material may be used as side panel extension material, such as when concurrently to forming the above discussed separation lines also pieces of the side panel extensions 1400 are separated, which can then be transferred to their correct positions, as indicated in Fig. 2P by a process which is discussed in more detail herein below.

For all these various executions it is important that the article comprises front and rear side panels which are unitary with or are connected to a single or composite material forming the leg hoop in the crotch region and, in the latter case, by having transition regions for connecting respective leg hoop regions with the side panels. These transition regions are formed by cut lines extending predominantly cross-directionally at the inward proximity of the side panels, i.e. inside of the side panels but in the proximity of the margins of the side panels towards the cross-directionally extending centre line. The transition regions are further delimited by separation lines extending between the side panels and the leg hoop crotch regions, such that at their end points towards the side panels are positioned laterally more inwardly, i.e. they extend from the proximity of the longitudinally extending side margins in the crotch region away therefrom and towards the longitudinally extending centre line, optionally ending there. The separation lines may be straight lines or curved. The effect of these transition regions it that upon opening the side panels and putting these into an in-use configuration, the leg hoop regions in the crotch region can be tensioned towards a tight fit around the legs whilst at the same time also the fit around the hips is appropriately adjusted.

Fig. 2 N and O show a schematic top- and cross-sectional view of an article according to the present invention. The further backsheet has been cut and severed to form an integral side panel in the form of a window 1170 of the further backsheet 1100, and been applied to the topsheet and/or backsheet. The composite is longitudinally overfolded along two parallel foldines 1175, each being positioned between the longitudinally extending centre line and side margins. Optionally side panel extensions 1400, which may be executed as extensible materials, may be applied. The laterally outwardly extending side margins of the side panels or side panel extension of the front and rear regions may now be positioned in an overlying position and may be connected such as by ultrasonic bonding and undergo final folding.

Fig 2B shows in addition to the further backsheet 1100, sidepanels 1200, namely two front 1212, 1213 and two rear side panels 1218, 1217 as may be cut from a single web side panel web 1210 in a chevron shape cut line 1214 and being longitudinally spaced apart, wherein the prime 1214' and double prime 1214" denote the front and rear margins of this separation line, respectively. Along the longitudinal centre line, the side panels are separated completely or partially, such as by a so-called perf'n pop connection 1224, such that upon use each a left and a right panel may be folded outwardly.

Fig. 2C depicts schematically a suitable manufacturing process to prepare the side panel pieces 1200 from a side panel web 1210 and to position these on the further backsheet 1100. Fig. 2D shows a top view of the materials as corresponding to the process steps of

Fig. 2C (with supply means not shown). First, the side panel web material 1210 is unwound from a web supply 2910. A longitudinal separation tool, such a cutting unit 2920 applies a longitudinal slit or perforation line 1224 along the longitudinal centre line of the web. A first CD-knife 2930 applies a cross-directional cut line 1219, which corresponds to the front 1219' and rear 1219" margins of the side panels. The material is spaced apart by the sum 1229 of the front 1229' and rear 1229" excess length, which is the length that the further backsheet 1100, once being cut into separate pieces, protrudes longitudinally beyond the end margins of the side panels in the front 1229' and in the rear 1229". Then a further separation is applied by a second separation tool, such as a cutting device 2940, thereby creating the chevron shaped cut 1214 with a front 1214' and rear 1214" margin. The front and the rear regions are spaced apart by the leg hoop spacing 1226, measured along the longitudinally extending side margin. The spaced apart side panel pieces may be supported by conventional support means, such as vacuum belts or a turret and the further backsheet 1100 is fed from a further backsheet supply unit 2900 at an overall repeating unit length that corresponds to the sum of the length of the front 1222 and rear 1228 side panel side margins plus the length of the leg hoop spacing plus the length of the front 1129' and rear 1229" further backsheet excess length.

Fig. 2B also shows schematically how the melt-fusion connecting between the further backsheet and the side panels can be achieved - for simplicity explained for one of the four connecting portions only. Preferably the connecting is achieved by using ultrasonic bonding with a flexible anvil, as described in the EP'434 application. Preferably, the bonding is made as a bonding line, wherein the term "bonding line" includes in the context of the present invention a series of bonding points along a line, but also the arrangement of bonding lines or points in a bonding region. The bonding line 1300 between the side panels and the further backsheet extends from the front 1219' or rear 1219" margins of the side panels towards the cross-directional centre line. The intersection with the front or rear margins is offset from the longitudinally extending side margins towards the longitudinal centre line and tapers towards these until it runs into their proximity over the unseparated strip 1129 between the first separation lines 1122ff and the longitudinally extending side margins of the further backsheet. This tapering of the bonding lines results in a darting effect during use, as described in more detail in the above referenced WO'275, to which express reference is made. On the side of the first separation line that is longitudinally positioned towards the cross-directional centre line, the bonding line runs essentially parallel the separation line, and stops shortly before intersecting with the second separation lines 1132ff. Similarly, as shown in Fig. 2K the side panels 1200 are shown as being executed with cross-directionally (and not chevron-shaped margins adapted to the cross-directionally extending first separation lines).

Optionally the side panels may be connected to side panel extensions, as indicated in Fig. 2E to 2F as well as in Fig. 2K. To this end, side panel extensions 1400 are provided as a single or twin web and positioned at least on the portion of the side panel web 1210 that corresponds to the rear side panels 1217, 1218 prior to separation, and symmetric to the longitudinal centre line. The side panel web and the side panel extension webs are now functionally connected along connecting lines 1410 to both sides of the longitudinal centre line by melt-fusion bonding, preferably by ultra-sonic bonding and more preferably by ultra-sonic bonding by using a flexible anvil as described in the above referenced application EP'434. Optionally, for the execution as depicted in Fig. 2L, to M, side panel extensions may be connected to the backsheet web in the front or back sidepanel region, or in both, prior to making first, second third and fourth cuts. The side panel extensions are cut and connected to the side panels, as described hereinabove. Optionally closure or fastening means 1450, preferably for a hook and loop connection, may be connected to the laterally outwardly oriented side margins of the side panel extensions 1400, preferably by ultra-sonic bonding and more preferably by ultra-sonic bonding by using a flexible anvil as described in the above referenced application EP'434.

The side panels 1200 together with the side panel extensions 1400 and closure means 1450, if present, are positioned longitudinally relative to the further backsheet at the respective further excess length 1229' and 1229" from the front and rear margin of the further backsheet, and such that they extend towards the cross-directional centre line, and further such that they reach longitudinally over the first separation lines in the further backsheet. If present, the side panel extensions and closure means are positioned such that they lie between the side panels and the further backsheet. In the embodiments depicted in Fig. 2M, L, the sidepanel extensions are preferably positioned between backsheet and the further backsheet.

Fig. 2 H and I depict schematically the relative arrangement of the resulting article 1000 with the further backsheet 1100 and side panels 1200 optionally with side panel extensions 1400 and fastening means 1450, with the absorbent pad 110 by the final article connection bond 1900. All these elements of the article are functionally connected by melt-fusion bonding, preferably by ultra-sonic bonding and more preferably by ultra-sonic bonding by using a flexible anvil as described in the above referenced application EP'434. In Fig 2I, the various connections are indicated by dotted lines whilst in Fig. 2H they are indicated by different symbols, for example, xx, yy or zz.

For an execution with third separation lines 1142 and 1143 as shown in Fig. 2J, these are accompanied by further connecting lines 1146 and 1147 as depicted in Fig. 2K by dotted lines and positioned laterally inwardly, i.e. towards the longitudinally extending centre line. The skilled person will readily realize that this connecting must not run into the side panels but stop at termination points 1148 positioned longitudinally inwardly, i.e. towards the cross-directionally extending centre line, of the side panels 1200.

Along the periphery of both the further backsheet and the absorbent pad, the connecting is functionally achieved by melt-fusion bonding 1900, preferably by ultra-sonic bonding and more preferably by ultra-sonic bonding by using a flexible anvil as described in the above referenced application EP'434, indicated by the symbol zz. The absorbent pad 110 comprises the bonding yy 175 connecting the absorbent core 120 to the backsheet 170 and the bonding xx 165, connecting the backsheet to the topsheet. The chassis subassembly comprises the bonding ww 1300 connecting the further backsheet 1100 to the side panel 1200 and the bonding oo 1410, connecting the side panels and the side panel extension. Optionally, the fixation means 1450 may be bonded to the side panel extension by bonding, which is not shown in Fig. 2I. The article 100 is now formed by bonding zz 1900 connecting the topsheet 160 and the further backsheet 1100 peripherally. The resulting article may now be further folded and packed for delivery to an end-user. Upon use, the end user folds the sidepanels outwardly to allow positioning around the waist of the wearer. This outward folding will - due to the special arrangement of the separation and connecting lines in the leg hoop region - form such a leg hoop around the upper thigh of a wearer as well as the body conforming fit around the buttocks of the wearer.

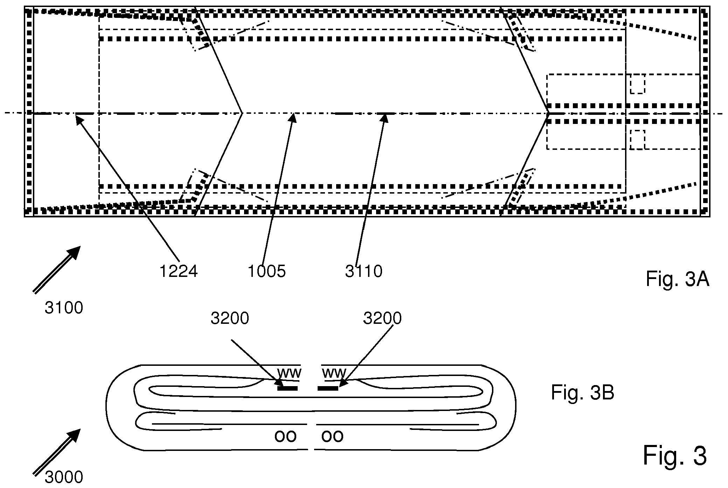

Optionally, the article may comprise a pocket in the crotch region for receiving bodily exudates such a faeces or urine. As indicated in Fig. 3, an article 3100 precursor (i.e. still in an unfolded configuration) as depicted in Fig 2H further comprises a topsheet opening in the topsheet in the crotch region, such as by applying a slit 3110 along the longitudinal centreline 1005, preferably before it is connected with the backsheet. After having combined the absorbent subassembly and the further backsheet / side panel subassembly, the longitudinal side margins of the precursor are overfolded towards the longitudinal centre line at least in the crotch region adjacent to the topsheet opening to form the final article 3000. The previously outwardly facing surface of the topsheet is now lying on itself and can be functionally connected to itself by tack down connection 3200 by applying a mechanical fastener patch, preferably by melt-fusion bonding, to the surface of the topsheet prior to combining it with the backsheet and the absorbent core. Upon application on a wearer, the same mechanism that forms the leg hoop as described hereinabove lifts the topsheet in the topsheet opening region away from the remainder of the article so as to allow bodily exudates to pass through. This mechanism and functionality is described in EP2424479A1 (Schmitz, C-4-S), to which express reference is made with regard to the functionality of such a "faeces trap".

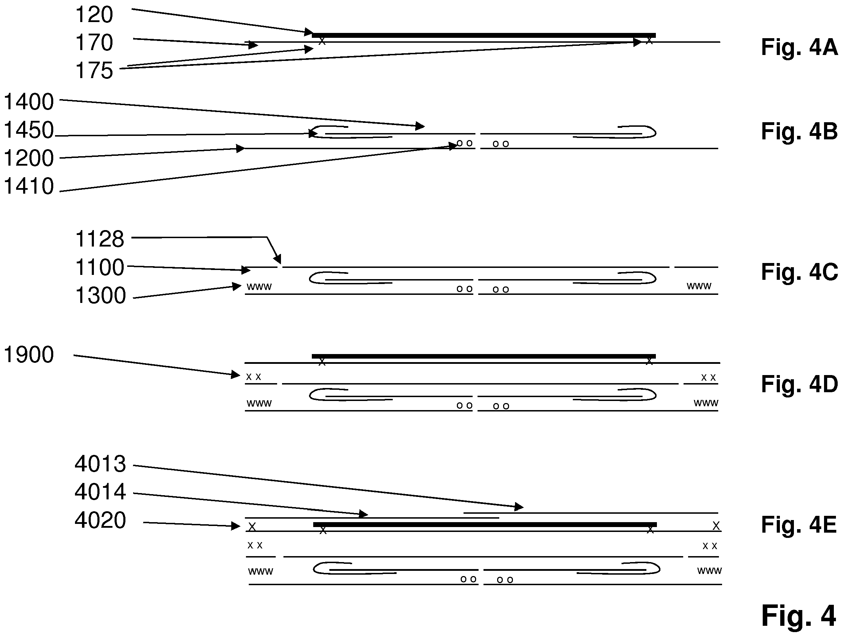

Referring now to Fig. 4A to H, a further execution of an article according to the present invention and the respective manufacturing process is described. As discussed in the context of Fig. 3, such an article comprises longitudinally overfolded side margins, as described in broader terms in WO2011/064272 (C-4-S; Schmitz) to which express reference is made with regard to such overfolding. Such an article may very suitably comprise openings on the wearer oriented side such that bodily discharges such as urine or faeces may penetrate there through.

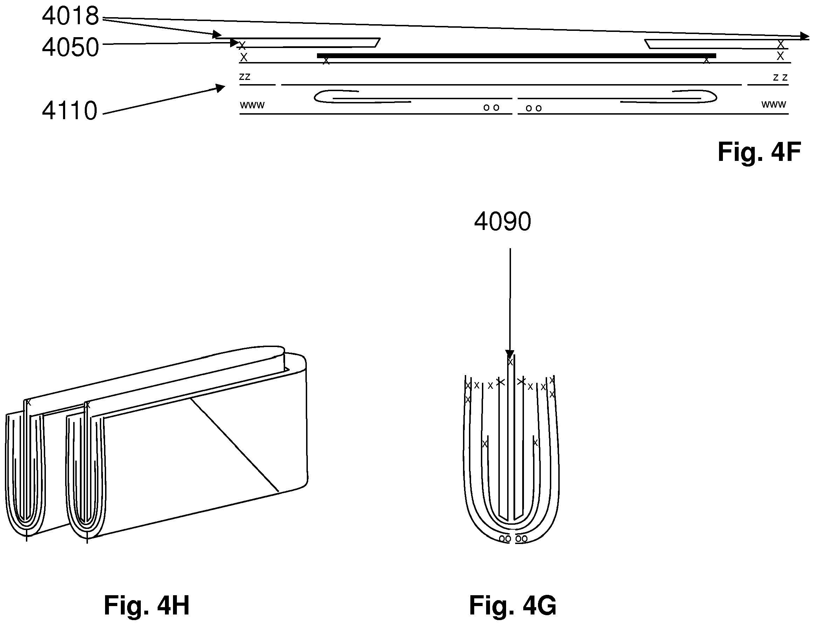

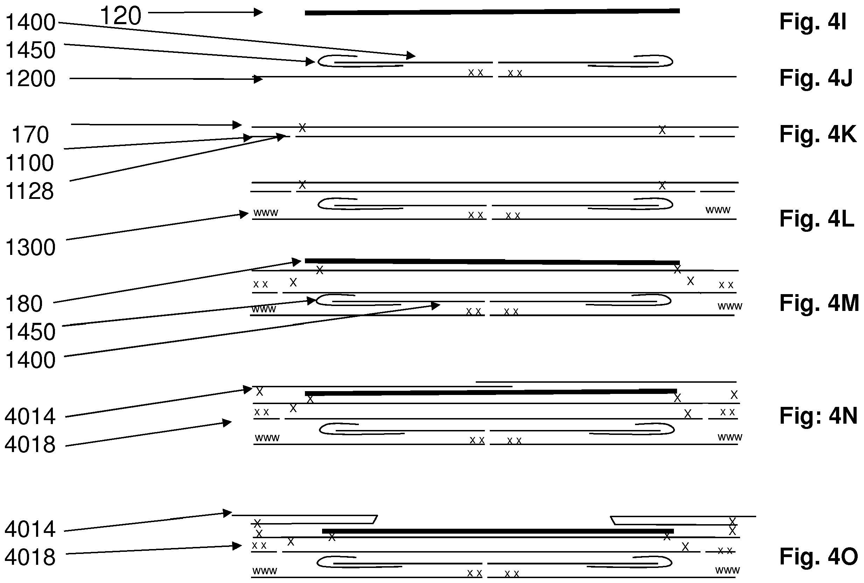

The Figures of Fig. 4 depict various subassemblies, as may be formed parallel to each other or consecutively. As shown in the figures the structures are essentially symmetric to a longitudinal centre line of the article and thus the description is made for one half only. As described hereinabove in the context if Fig. 2, and shown in Fig 4 A to 4 G in a cross-sectional view parallel to a cross-directional centreline, but positioned in the rear portion of the article, a cut piece of an absorbent core 120 is formed, positioned on a further backsheet 1100 and connected by connecting lines 175, preferably ultrasonic connecting lines. The further backsheet 1100 may then be cut to its desired length. Further, there are provided cut pieces of side panel materials 1200, optionally connected by connecting lines 1410 to side panel extension 1400, optionally comprising fixation means 1450. As discussed in the above, and further shown in Fig. 4C, the backsheet material 170 such as nonwoven fabric and comprising leg hoop slits, of which the predominantly machine directionally oriented part 1128 is shown, is positioned on the subassembly of Fig. 4B. The resulting subassembly is further combined with the subassembly as shown in Fig. 4A and connected by construction bond 1900, preferably by ultrasonic bonding. In such an embodiment, either or both of the backsheet 170 and the further backsheet 1100 may be liquid impermeable, but preferably are vapour permeable.

The articles as depicted in Fig 4 further comprise a longitudinally split topsheet comprising a strip for each the right 4013 and left 4014 portion of the article - refer to Fig. 4E. Each strip is connected to the subassembly of Fig. 4B by a topsheet connecting bond 4020, preferably ultrasonically, at its outer longitudinal perimeter, whilst the two topsheet portions overlap in the proximity of the longitudinally extending centre line. Then, the unbonded portions of the topsheet portions are lifted off, preferably by vacuum or mechanical gripping means, and outwardly overfolded, preferably around a folding rail, such that they extend laterally outwardly of the perimeter of the subassembly (see 4018. Then, the total subassembly of Fig. 4F is overfolded along the longitudinally extending centreline, such that the portions 4018 overlay each other and can readily be connected, such as by final topsheet bonding 4090 - see Fig. 4G (with side panel extensions and tapes omitted for ease of reference). If an article comprising a pocket in the crotch region for receiving bodily exudates such a faeces or urine as described in the above in the context of Fig. 3 is desired, the connecting of the topsheet halves is not executed in art least a portion of the crotch area, thereby providing a urine or faeces passage.

Optionally, the final article can now be folded around its cross-directionally extending centre line, resulting in a rectangularly folded piece (see Fig. 4H), which can easily be packed, shows a pleasing appearance, and is easy to use for the consumer because the longitudinal fold is already provided in the packed article. The opening of the article for the use can readily be accomplished. Yet a further approach to arrive at essentially the same product as shown in Fig. 4G and 4H is explained by referring to Fig. 4 I to O. In this case, a core subassembly 180 is formed (see Fig. 4I), which may comprise an absorbent core, a carrier and a cover material as described in the above. A side panel subassembly is formed (see Fig. 4J), comprising side panel extension 1400 comprising closure means 1450 and being connected to side panel 1200. A backsheet 170 is combined with a further backsheet 1100, the latter comprising separation lines, here shown as 1128. The sidepanel subassembly as shown in Fig. 4J is now connected to the combined backsheets, see Fig. 4L by backsheet-to-sidepanel connection 1300. As indicated in Fig. 4M, the absorbent core subassembly 180 is connected to the backsheet 170 laterally or longitudinally outside of the side panel extensions 1400 or fixation means 1450. In analogy to Fig. 4E and 4F, the topsheet stripe 4014 is added and connected in the proximity of the lateral outward edge 4018 (see Fig. 4N), folded outwardly over lateral outward edge 4018 and connected to itself inward of the lateral outward edge. The resulting structure is essentially the same as resulting from the process steps as described in the above in the context of Fig. 4G, and can be folded as shown in Fig. 4H. It should be noted that also for this execution the preferred method of connecting for all steps is ultrasonic bonding.

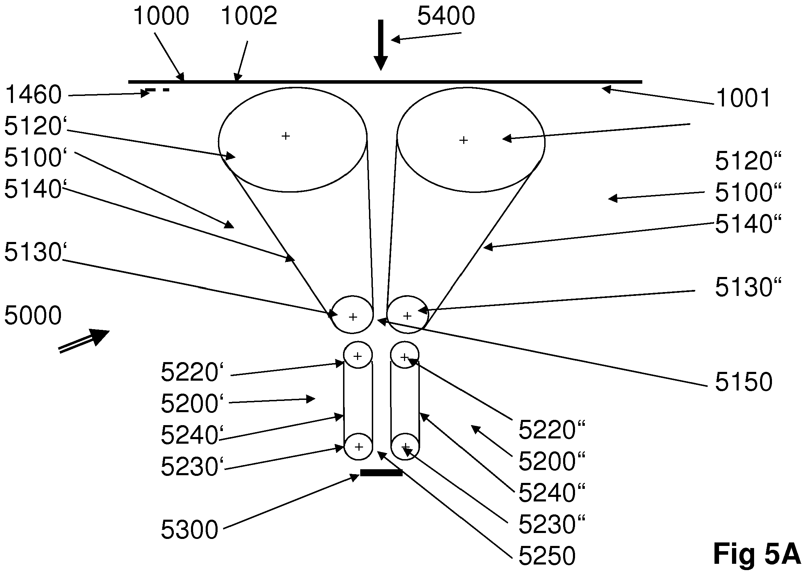

Yet a further execution for forming an essentially melt-fusion bonded article is presented in Fig. 5 A to H, namely a so called pre-closed pants-style article, which may be manufactured and donned in the form of pants yet having the possibility of being opened and re-closed by re-usable fasteners.

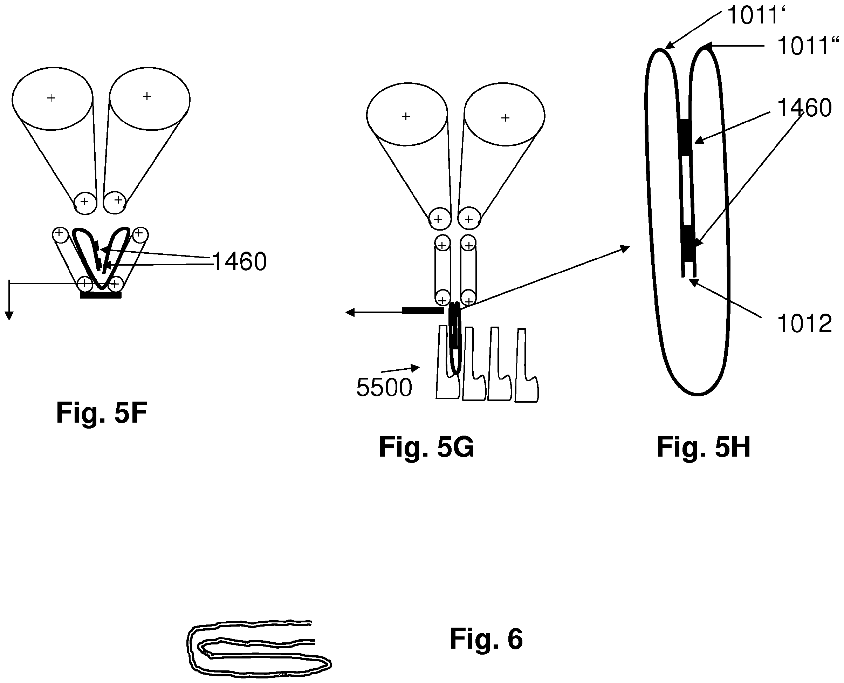

It is an important feature of such an article and its manufacturing method that it is folded along cross-directionally extending fold lines in a particular way, here referred to as "inward quad fold". Conventionally, longitudinally "quad folded" articles are folded twice, a first time along the cross-directionally extending centre line, thus halving the longitudinal extension, and a second time along the cross-directionally extending centre line of the folded article, thusly forming four layers (see Fig 6).

In contrast to this prior art design, the inward quad fold has a first fold line 1010 in the vicinity of the cd centreline of the article, and two secondary fold lines, one of them 1011" placed in the leading half of the article, the other one 1011 in the trailing half, both secondary fold lines arranged such that they fold respective portions of the article inwardly. The process of the "inward quad-folding" employs web-handling equipment as described in more detail in WO2006/103487, to which express reference is made.

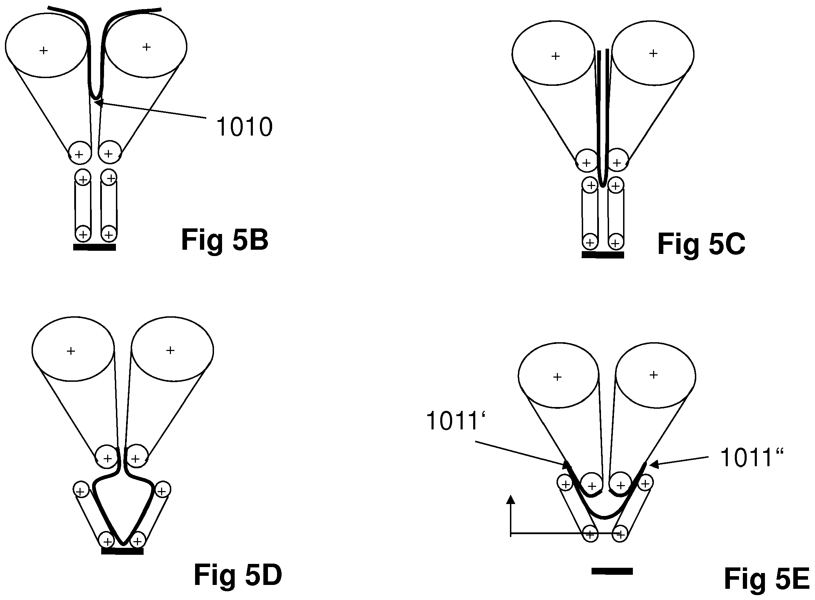

As indicated in Fig. 5, the inward quad folding can be performed on an inward quad folding means 5000, comprising a first pair 5100 of web support means 5100' and 5100" and a second pair 5200 of web support means 5200' and 5200", each of the web support means comprising an essentially endless surface 5140', 5140", 5240', 5240", preferably a belt, and a first 5120', 5120", 5220', 5220" and a second 5130', 5130", 5230', 5230" guide means. At least one of the guide means for each web support means comprises a drive means, preferably an essentially freely programmable drive means, such as an electrical servo motor. Each of these elements is described in more detail in the above referenced WO'487.

For the present execution, the two first web support means 5100' and 5100" are arranged relative to each other such that a first gap 5150 is formed between them, adapted to receive web materials. Following the direction of the gap over and beyond the other of the guide means 5130', 5130", a further gap 5250 is formed by and between the second web support means 5200' and 5200. The second web support means 5200' and 5200" are arranged such that they can be pivoted around the guide means 5230' and 5230" that are positioned away from the first web support means or that the distance between 5220' and 5220" can be varied, such that the second web support means can be brought from an essentially parallel arrangement to an angled arrangement. Further, the first and the second web handling means can be moved relative to each other along the direction of the first gap. The equipment may further comprise a moveably mounted stopper means 5300, which can be positioned at a location essentially at the end of gap 5250, formed between the second web support means 5200' and 5200" if these are in an essentially parallel arrangement, and which can be moved away from this position. This stopper means ensures reproducible positioning of the article. Alternatively, this precise positioning may be achieved by tightly controlled stopping of support means 5200', 5200".

The equipment may further comprise a tucker means 5400 (shown in Fig. 5A only) and a means for receiving the inwardly quad folded article of piece of material 5500 (shown in Fig. 5G only).

If appropriate measures for laterally removing the articles are taken, the equipment 5000 may correspond to a web handling section (in the terminology of WO'487) and be a part of a web path splitting means.

The process of creating the inward quad folded material or article on such an equipment is for simplicity of the explanation made by referring in Fig. 5 to a piece of web material or an article 1000, exhibiting a first and a second surface. For example, the first surface 1001 may represent the garment oriented or backsheet surface of an article and the second surface 1002 may represent the wearer oriented or topsheet surface of an article, and further subassemblies or elements may be comprised between these surfaces.

The web material 1000 is fed tangentially to guide means 5120' and 5120" of the first web support means 5100 - see Fig. 5A, optionally guided by a further web support means (not shown) with its first surface 1001 oriented towards the web support means. Upon appropriate movement control of the guide means, optionally supported by action of the tucker means 5400, the article is fed into the first gap such that a line parallel to the cross-directional centre line forms the leading edge 1010 for the movement through the first gap, thusly forming a "bifolded" article. The second surface 1002 is now folded onto itself. With the second web support means 5200' and 5200" positioned such that the second gap 5250 between them is aligned with the first gap, the bi-folded article can be moved through the second gap until stopped by the stopper plate 5300, or by accurately stopping the drive means of 5200', 5200". Then the guide means positioned away from the stopper plate 5220', 5220" are moved away from each other (Fig. 5D) whilst the article or web remains attached thereto. Further, the distance between the first and the second web support means may be reduced, such as by moving the second web support means in the direction towards the first one. In this period, the now trailing portions of the material (corresponding to the leading and trailing edge of the article before being tucked into the first gap) remain attached to the first web support means such that on either side a further fold 1011' and 1011" is formed, as indicated in Fig. 5E. Then, the web or material is released from the first web support means, and the distance between the first and the second web support means is increased again to allow again for a parallel positioning of the web support means 5200' and 5200" (Fig. 5F). Removing the stopper plate 5300 out of the path, the inward quad folded article can be moved to the receiving means 5500, such as for further processing such as packaging.

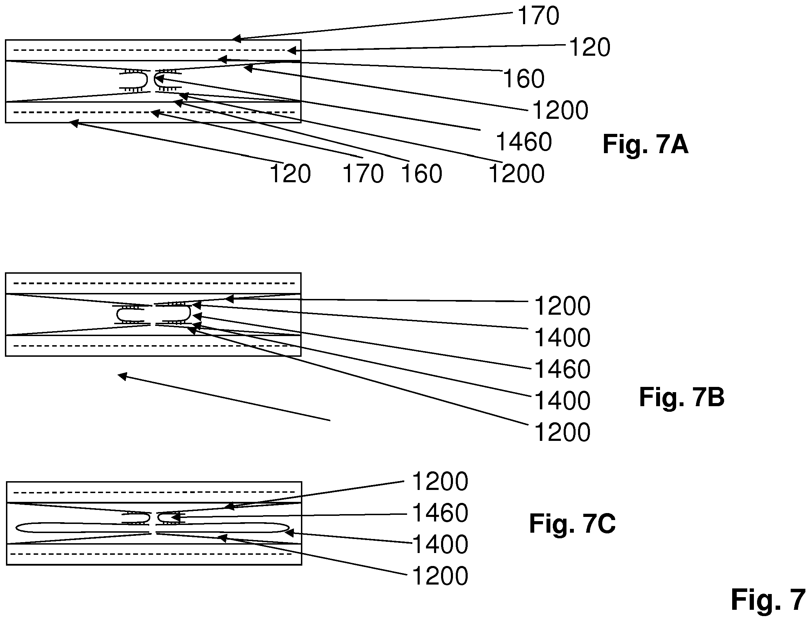

This inward quad folding can now be very suitably employed to form so called pre-closed articles. This refers to articles to be worn on the lower torso of a wearer, such as pants or diapers. However, the closure of the pants shape may be formed by recloseable means, such as well-known mechanical fastening systems, also referred to as hook-and-loop means, such as traded under the trademark VELCRO ®. If the article is positioned such that the second or topsheet surface corresponds to the surface oriented towards the wearer during use, and the first or backsheet surface faces away from the wearer, closure means 1460, such as folded hook patches 1460 in Fig. 7 exhibiting hooks on both opposite surfaces, may be placed on the first or backsheet surface (or alternatively on the second or topsheet surface, on products with one or two single layer extensions per side) prior to the delivery to the present process (indicated in Fig. 5A, but omitted for 5B to 5E). In the process step as shown in Fig. 5G, the second web support means compress the folded material or article, thereby forcing the hooks to also engage with the other surface and the closed pants structure is formed. Prior to donning, the waist portions 1012 can be readily pulled out directly by user, such as be inserting a hand between the second folds 1011' and 1011" and grasping and subsequently pulling out the waist edges 1012. Optionally pulling aids such as a strip connected to first surface a extending from the waist edges to the second fold lines can be inserted. Optionally, visual guide means such as printing onto the first surface may aid the user.

In Fig. 7, variants for such an execution are depicted. Fig 7A depicts a design with side panels 1200 but without side panel extensions and with the fold line of the hook patches being oriented towards the longitudinal centreline of the article. Fig. 7B shows an execution with each one side panel extension 1400 being releasably connected to the hook patches 1460. In Fig. 7C a single but overfolded side panel extension 1400 is connected to one side of the hook patch 1460, whilst the other side is connected to the respective side panel.

## Claims

1. A disposable absorbent article to be worn on the lower torso of a wearer comprising a crotch region, to be positioned in the crotch region of a wearer during use and a front and a rear waist region extending forwardly and rearwardly to the waist region of a wearer during use,
the article comprising
a liquid permeable topsheet;
a liquid retaining backsheet;
optionally a further backsheet;
at least a first and a second side panel;
a closing element for connecting the front and rear waist regions, a liquid absorbing core
comprising
absorbent, preferably superabsorbent, material, preferably in particulate form, further preferably comprising a first and a second enveloping web;
positioned between said topsheet and said backsheet;
**characterized in that** melt-fusion bonds functionally connect
the topsheet, the backsheet and the absorbent core to each other, and
said first and second side panels to both said topsheet and backsheet,
and wherein further said melt-fusion bonds are essentially free of adhesive.

2. A disposable article according to claim 1,
wherein said liquid absorbent core comprises a first and a second envelope webs
which are functionally connected to each other by melt-fusion bonds.

3. A disposable absorbent article according to any of claims 1 to 2,
further comprising one or more further web material(s) that is / are functionally connected to said topsheet, backsheet or core by melt-fusion bonds, wherein
the further web material(s) is / are selected from the group consisting of
liquid handling webs, preferably selected from the group consisting of
- liquid acquisition materials exhibiting an open pore structure and preferably comprising resilient fibres, more preferably comprising polyester fibres;
- and liquid distribution materials preferably comprising cellulosic or melt-blown
fibres;
a further topsheet;
side panel extensions;
leg hoops;
barrier cuffs.

4. An absorbent article according to any of the preceding claims,
comprising construction bonds for establishing the integrity of the article and optionally application bonds for establishing or maintaining the positioning of the article on a wearer, wherein at least the construction bonds are only melt-fusion bonds.

5. An article according to any of the preceding claims,
the article being an article to be worn on the lower torso of a wearer, preferably being selected from the group consisting of an open diaper, a preclosed and reopenable pants-style article or a closed pants style article, for babies, toddlers or adults.

6. An article according to any of the preceding claims being inwardly quad folded.

7. A method for the manufacturing of an absorbent article to be worn on the lower torso of babies, toddlers or adults,
the article being selected from the group consisting of an open diaper, a preclosed and reopenable pants-style article or a closed pants style article,
the article exhibiting
x-, y-, and z-directional extension;
longitudinal centre line;
front and rear waist regions, each comprising cross-directionally a first and a second region, and a crotch region;
cross-directional centre line in the crotch region,
the method being **characterized by** providing individual pieces of an absorbent core manufactured by
a) providing
a1)
- absorbent, preferably superabsorbent material, preferably in particulate form,
- a first and a second enveloping web;
- optionally a further web material for enhanced liquid handling;
a2) backsheet web material exhibiting two longitudinally extending side margins;
a3i) topsheet web material;
and side panel material;
b) preparing individual pieces of absorbent cores by
b1) positioning the absorbent material between the first and the second enveloping web;
b2) functionally connecting the first and the second enveloping web material;
b3) separating the connected web material to form individual pieces of absorbent cores;
further comprising the steps of
m) providing
m1) a further backsheet web material, exhibiting a longitudinal centre line which essentially coincides with the longitudinal centre line of the article and longitudinally extending side margins, optionally being composed of sub-webs;
m3) optionally side panel extension web materials;
m4) optionally closure tapes;
n) preparing a chassis sub-assembly from the further backsheet,
optionally combined with the side panel web materials and/or the side panel extension web materials,
by applying separation lines to the further backsheet,
which extend predominantly in the MD and symmetrically to a longitudinally extending centre line of the further backsheet, whereby either
n1) leg hoops are formed by the further backsheet material,
whilst side panel materials and optionally side panel extension materials are separated from side panel web materials or side panel extension web materials into pieces and added and functionally connected thereto, whereby the separation line
is positioned at least in a portion of the crotch region less than 9 cm, preferably less than 3 cm, but more than 0 cm away from the longitudinally extending side margins of the further backsheet material and is intersecting a further separation line, which extends predominantly cross-directionally from the point of intersection towards but not extending into the side margin of the further backsheet material;
or
n2) side panels and leg hoops are formed by the further backsheet material and optionally side panel extension materials are separated from a side panel extension web material into pieces and added and functionally connected to the side panel of the further backsheet,
whereby the separation line is positioned
at least in a portion of the crotch region less than 9 cm, preferably less than 3 cm, but more than 0 cm away from the longitudinally extending side margins of the further backsheet,
and being less than 9 cm, preferably less than 3 cm, more preferably less than 1 cm away from and including coinciding with the longitudinal centre line in the front and rear waist region, optionally being executed as a perf'n pop separation line;
or
n3) side panel extensions, side panels and leg hoops are formed by the further backsheet material, whereby the side panel extensions are separated from the further backsheet material, thereby forming sets of side panel extension pieces,
whereby the separation line is positioned
at least in a portion of the crotch region less than 9 cm, preferably less than 3 cm, but more than 0 cm away from the longitudinally extending side margins of the further backsheet,
and being less than 9 cm, preferably less than 3 cm, more preferably less than 1 cm away from and including coinciding with the longitudinal centre line in the front and rear waist region,
optionally being executed as a perf'n pop separation line;
o) combining the chassis sub-assembly with the individual pieces of an absorbent core and the backsheet and topsheet, thereby repositioning the sets of side panel extension pieces, when/if present, machine directionally relative to the remaining further backsheet material such that
o1) the backsheet and topsheet envelope the absorbent core; and
o2) the further backsheet is positioned on the backsheet on the opposite side of the absorbent core; and
o3) the optional side panel extensions are positioned between the further backsheet and the backsheet;
p) functionally connecting
p1) the further backsheet to the backsheet and/or the topsheet;
p2) if present, the side panel material pieces to the further backsheet and/or the topsheet;
p3) the side panels as integral parts of the further backsheet to the backsheet and/ or the topsheet in the proximity of the longitudinal side margins;
p4) if present, the side panel extension pieces to the side panels;
p5) optionally the absorbent core to the topsheet and/or backsheet;
q) providing a closing element for the article by
q1) adding and connecting a closure tape for the making of an open diaper or for an pre-closed and reopenable diaper; or
q2) positioning the side margins of the front and rear portions of the article in an abutting or overlapping mode for forming a pants style article and connecting the portions;
z) separating a full assembly from the continuous composite so as to form individual articles and optionally further processing such articles,
wherein the functional connecting in the steps b2), c2), c3), p1), p2), p3), p5), q) and p4) is executed by applying energy, preferably thermal energy, more preferably sonic energy and most preferably ultrasonic energy to establish melt-fusion bonds which are essentially free of adhesive.

8. A method for the manufacturing of a disposable absorbent article according to claim 7, wherein
a3ii) the process step a3) of providing the topsheet comprises the providing a first and a second strip of topsheet web material, each of the strips exhibiting a cross-directional width of more than half the width of the further backsheet web material,
the process further comprising the steps of
r1) positioning the first and second strip of topsheet web material such that their laterally outward side margin is essentially in registry with the respective laterally outward margins of the further backsheet and such that the opposite margins of the strips extend beyond the longitudinally extending centreline of the article, such that the strips overlap in the proximity of the longitudinally extending centreline;
r2) functionally connecting the strips of topsheet material with the further backsheet material in the proximity of and along the longitudinally extending side margins;
r3) overfolding the opposite side margin of the strip of topsheet material outwardly over and beyond the connecting of the strip of topsheet material to the further backsheet;
r4) functionally connecting the overfolded portions of the strips of topsheet material to the underlying portion that is already connected to the further backsheet;
r5) folding the resulting subassembly along the longitudinal centreline such that the overfolded portions of the first and the second strip are positioned towards each other and the portions of said strips extend beyond the further backsheet;
r6) functionally connecting the portions of the first and the second strip, that extend beyond the connecting to the further backsheet, to each other;
wherein the functional connecting in steps r2), r4), and r6) is executed by applying energy, preferably thermal energy, more preferably sonic energy and most preferably ultrasonic energy to establish melt-fusion bonds.

9. A method for manufacturing a disposable absorbent article according to any of claims 7 to 8, further comprising the steps of applying at least one further web material selected from the group consisting of
t1) liquid handling webs, preferably selected from the group consisting of
- liquid acquisition materials exhibiting an open pore structure and preferably comprising resilient fibres, more preferably comprising polyester fibres;
- liquid distribution materials preferably comprising cellulosic or melt-blown fibres;
t2) a further topsheet;
t3) side panel extensions;
t4) leg hoops;
t5) barrier cuffs,
wherein the further web material(s) is/are functionally connecting by applying energy, preferably thermal energy, more preferably sonic energy and most preferably ultrasonic energy to establish melt-fusion bonds.

## Patentansprüche

1. Saugfähiger Wegwerfartikel zum Tragen am Unterleib eines Trägers,
mit einem Schrittbereich, der während des Gebrauchs im Schrittbereich eines Trägers positioniert werden soll und einem vorderen und einem hinteren Taillenbereich, die sich während des Gebrauchs vorwärts und rückwärts zum Taillenbereich eines Trägers erstrecken,
wobei der Artikel umfasst
eine flüssigkeitsdurchlässige Oberlage;
eine flüssigkeitsrückhaltende Unterlage;
optional eine zusätzliche Unterlage;
mindestens ein erstes und ein zweites Seitenteil;
ein Verschlusselement zum Verbinden des vorderen und hinteren Taillenbereichs;
einen zwischen der Oberlage und der Unterlage angeordneten flüssigkeitsabsorbierenden Kern, umfassend absorbierendes, vorzugsweise superabsorbierendes Material, vorzugsweise in Partikelform, bevorzugt umfassend eine erste und eine zweite Umhüllungsbahn;
**dadurch gekennzeichnet, dass** Schmelzverbindungen
- die Oberlage, die Unterlage und der flüssigkeitsabsorbierende Kern miteinander,
- sowie die ersten und zweiten Seitenteile sowohl mit der Oberlage wie mit der Unterlage funktionell verbinden,
und weiterhin diese Schmelzverbindungen im Wesentlichen leimfrei sind.

2. Saugfähiger Wegwerfartikel nach Anspruch 1,
wobei der flüssigkeitsabsorbierende Kern eine erste und eine zweite Umhüllungsbahn umfasst, die miteinander durch Schmelzverbindungen funktionell verbunden sind.

3. Saugfähiger Wegwerfartikel nach einem der Ansprüche 1 bis 2,
der ferner ein oder mehrere zusätzliche Bahnmaterialien umfasst, welche/s funktionell mit der Oberlage, der Unterlage oder dem Kern durch Schmelzverbindungen verbunden sind,
wobei
das / die zusätzliche/n Bahnmaterial /ien ausgewählt ist / sind aus der Gruppe bestehend aus
- Flüssigkeitshandhabungsbahnmaterialien, bevorzugt ausgewählt aus der Gruppe bestehend aus
- Flüssigkeitsaufnahmematerialien, die eine offenporiger Struktur aufweisen und bevorzugt rückstellfähigen Fasern, besonders bevorzugt Polyesterfasern, umfassen;
- und Flüssigkeitsverteilungsmaterialien, die vorzugsweise Zellulosefasern oder schmelzgeblasene Fasern umfassen;
eine zusätzliche Oberlage;
S eitenteilverlängerungen;
Beinbändchen;
Ab dichtungsbündchen.

4. Saugfähiger Wegwerfartikel nach einem der vorherigen Ansprüche,
umfassend konstruktive Verbindungen zur Schaffung der strukturellen Integrität des Artikels sowie optional Anwendungsverbindungen zum Anlegen und Aufrechterhalten der Positionierung des Artikels an einem Träger, wobei zumindest die konstruktiven Verbindungen nur Schmelzverbindungen sind.

5. Ein Artikel gemäß einem der vorherigen Ansprüche, zum Tragen am Unterleib eines Trägers,
wobei der Artikel vorzugsweise aus der Gruppe ausgewählt ist, die aus einer offenen Windel, einem vorverschlossenen und wieder zu öffnenden Artikel im Hosenstil oder einem geschlossenem Artikel im Hosenstil besteht, für Babys, Kleinkinder oder Erwachsene.

6. Ein Artikel gemäß einem der vorherigen Ansprüche, der vierfach nach innen gefaltet ist.

7. Verfahren zur Herstellung eines absorbierenden Artikels, zum Tragen am Unterleib von Babys, Kleinkindern oder Erwachsenen,
wobei der Artikel aus der Gruppe ausgewählt ist, die aus einer offenen Windel, einem vorverschlossenen und wieder zu öffnenden Artikel im Hosenstil oder einem geschlossenen Artikel im Hosenstil besteht,
wobei der Artikel
eine Ausdehnung in x-, y-, z- Richtungen aufweist, sowie
eine Längsmittellinie und
vordere und hintere Taillenbereiche, die jeweils in Querrichtung einen ersten und einen zweiten Bereich und einen Schrittbereich umfassen,
eine Mittellinie in Querrichtung im Schrittbereich,
wobei das Verfahren **dadurch gekennzeichnet ist, dass** einzelne Stücke eines absorbierenden Kerns bereitgestellt werden, mit
a) dem Bereitstellen von
a1) - absorbierendem, vorzugsweise superabsorbierendem Material, vorzugsweise in Partikelform,
- einer ersten und einer zweiten Umhüllungsbahn;
- optional ein zusätzliches Bahnmaterial für eine verbesserte Flüssigkeitshandhabung;
a2) Unterlagenbahnmaterial mit zwei sich in Längsrichtung erstreckenden Seitenrändern;
a3i) Oberlagenbahnmaterial;
und Seitenteilmaterial;
b) dem Vorbereiten einzelner Stücke von absorbierenden Kernen durch
b1) Positionieren des absorbierenden Materials zwischen der ersten und der zweiten Umhüllungsbahn;
b2) funktionelles Verbinden der ersten und der zweiten Umhüllungsbahn;
b3) Trennen des verbundenen Bahnmaterials, um einzelne Stücke absorbierender Kerne zu bilden; ferner umfassend die Schritte
m) Bereitstellen von
m1) einem zusätzlichen Unterlagenbahnmaterial mit einer Längsmittellinie, die im Wesentlichen mit der Längsmittellinie des Artikels zusammenfällt und mit sich in Längsrichtung erstreckenden Seitenrändern, welches gegebenenfalls aus Teilbahnen besteht;
m3) optionalen Seitenteilverlängerungsbahnmaterialien;
m4) gegebenenfalls einem Verschlussband;
n) Herstellen einer Chassis-Baugruppe aus der zusätzlichen Unterlage, gegebenenfalls kombiniert mit den Seitenteilbahnmaterialien und / oder den Seitenteilverlängerungsbahnmaterialien, indem die zusätzliche Unterlage mit Trennlinien versehen wird, die sich überwiegend in MD und symmetrisch zu einer längs verlaufenden Mittellinie der zusätzlichen Unterlage erstrecken, wobei entweder
n1) Beinbändchen durch das zusätzliche Unterlagematerial gebildet werden, während Seitenteilmaterialien und gegebenenfalls Seitenteilverlängerungsmaterialien von Seitenteilbahnmaterialien oder Seitenteilverlängerungsbahnmaterialien in Stücke getrennt und hinzugefügt werden und funktionell damit verbunden werden, wobei die Trennlinie zumindest in einem Teil des Schrittbereichs angeordnet ist, der weniger als 9 cm, vorzugsweise weniger als 3 cm, aber mehr als 0 cm von den sich in Längsrichtung erstreckenden Seitenrändern des zusätzlichen Unterlagenmaterials entfernt ist und einen Schnittpunkt hat mit einer zusätzlichen Trennlinie, die sich vorwiegend in Querrichtung vom Schnittpunkt in Richtung auf aber nicht in den Seitenrand des zusätzlichen Unterlagenmaterials erstreckt;
oder
n2) Seitenteile und Beinbändchen durch das zusätzliche Unterlagenbahnmaterial gebildet werden und gegebenenfalls Seitenteilverlängerungsmaterialien von einem Seitenteilverlängerungsbahnmaterial in Stücke getrennt und hinzugefügt und funktionell mit dem Seitenteil des zusätzlichen Unterlagenbahnmaterials verbunden werden, wodurch die Trennlinie zumindest in einem Abschnitt des Schrittbereichs angeordnet ist, der weniger als 9 cm, vorzugsweise weniger als 3 cm, aber mehr als 0 cm von den sich in Längsrichtung erstreckenden Seitenrändern der zusätzlichen Unterlage entfernt ist und weniger als 9 cm, vorzugsweise weniger als 3 cm, mehr bevorzugt weniger als 1 cm von der Längsmittellinie im vorderen und hinteren Taillenbereich entfernt und gegebenenfalls mit dieser zusammenfallend,
gegebenenfalls als Perfn-Pop-Trennlinie ausgeführt;
oder
n3) Seitenteilverlängerungen, Seitenteile und Beinbändchen durch das zusätzliche Unterlagenmaterial gebildet werden, wobei die Seitenteilverlängerungen von dem zusätzlichen Unterlagenmaterial getrennt werden, wodurch Gruppen von Seitenteilverlängerungsstücken gebildet werden, wobei die Trennlinie zumindest in einem Teil des Schrittbereichs weniger als 9 cm, vorzugsweise weniger als 3 cm, aber mehr als 0 cm von den sich in Längsrichtung erstreckenden Seitenrändern der zusätzlichen Unterlage entfernt positioniert wird, und weniger als 9 cm, vorzugsweise weniger als 3 cm, bevorzugter weniger als 1 cm von der Längsmittellinie im vorderen und hinteren Taillenbereich entfernt und gegebenenfalls auch mit dieser zusammenfallend,
optional als Perfn Pop-Trennlinie ausgeführt;
o) Kombinieren einer Chassis-Unterbaugruppe mit den einzelnen Stücken eines absorbierenden Kerns und der Unterlage und der Oberlage,
wobei die Gruppen von Seitenteilverlängerungsstücken, wenn / falls vorhanden, in Bezug auf das verbleibende zusätzliche Unterlagenmaterial in Maschinenrichtung neu positioniert werden, sodass
o1) die Unterlage und die Oberlage den absorbierenden Kern umhüllen; und
o2) die zusätzliche Unterlage auf der Unterlage auf der gegenüberliegenden Seite des absorbierenden Kerns positioniert ist; und
o3) die optionalen Seitenteilverlängerungen zwischen der zusätzlichen Unterlage und der Unterlage positioniert sind;
p) funktionelles Verbinden von
p1) der zusätzlichen Unterlage mit der Unterlage und / oder der Oberlage;
p2) falls vorhanden, die Seitenteilmaterialstückemit der zusätzlichen Unterlage und / oder der Oberlage;
p3) die Seitenteile als integraler Bestandteil der zusätzlichen Unterlage mit der Unterlage und / oder der Oberlage in der Nähe der Längsseitenränder;
p4) falls vorhanden, die Seitenteilverlängerungsstücke mit den Seitenteilen;
p5) gegebenenfalls den absorbierenden Kern mit der Oberlage und / oder der Unterlage;
q) Bereitstellen eines Verschlusselements für den Artikel durch
q1) Hinzufügen und Verbinden eines Verschlussbands zum Herstellen einer offenen Windel oder einer vorverschlossenen und wieder zu öffnenden Windel; oder
q2) Positionieren der Seitenränder der vorderen und hinteren Bereiche des Artikels in einer Stoßpositionierung oder einem überlappenden Modus zum Bilden eines Artikels im Hosenstil und Verbinden der Teile;
z) Trennen einer vollständigen Baugruppe von dem Endlosverbund zu Einzelartikeln und gegebenenfalls Weiterverarbeiten derartiger Artikel,
wobei die funktionelle Verbindung zumindest in den Schritten b2), c2), c3), p1), p2), p3), p5), q) und p4) durch Anwenden von Energie, vorzugsweise Wärmeenergie, bevorzugterweise Schallenergie und am bevorzugtesten Ultraschallenergie, ausgeführt wird, um Schmelzschmelzbindungen herzustellen,
die im Wesentlichen leimfrei sind.

8. Verfahren zur Herstellung eines saugfähigen Wegwerfartikels nach Anspruch 7, wobei a3ii) der Verfahrensschritt a3) des Bereitstellens der Oberlage das Bereitstellen eines ersten und eines zweiten Streifens von Oberlagenbahnmaterial umfasst,
wobei jeder der Streifen in Querrichtung eine Breite von mehr als der Hälfte der Breite des zusätzlichen Unterlagenbahnmaterials aufweist,
wobei das Verfahren ferner die Schritte umfasst:
r1) Positionieren des ersten und zweiten Streifens des Oberlagenbahnmaterials derart, dass der seitlich nach außen gerichtete Seitenrand im Wesentlichen mit dem jeweils seitlich nach außen gerichteten Rand der zusätzlichen Unterlage übereinstimmt derart, dass sich die gegenüberliegenden Ränder der Streifen über die sich in Längsrichtung erstreckende Mittellinie des Artikels hinaus erstrecken,
so dass sich die Streifen in der Nähe der sich in Längsrichtung erstreckenden Mittellinie überlappen;
r2) funktionelles Verbinden der Streifen des Oberlagenmaterials mit dem zusätzlichen Unterlagenmaterial in der Nähe und entlang der sich in Längsrichtung erstreckenden Seitenränder;
r3) Überfalten des gegenüberliegenden Seitenrands des Oberlagenmaterialstreifens nach außen über die Verbindung des Oberlagenmaterialstreifens mit der zusätzlichen Unterlage hinaus;
r4) funktionelles Verbinden der überfalteten Abschnitte der Streifen des Oberlagenmaterials mit dem darunterliegenden Teilbereich, der bereits mit der zusätzlichen Unterlage verbunden ist;
r5) Falten der resultierenden Unterbaugruppe entlang der Längsmittellinie, so dass die überfalteten Abschnitte des ersten und des zweiten Streifens zueinander positioniert sind und sich die Abschnitte der Streifen über die zusätzliche Unterlage hinaus erstrecken;
r6) funktionelles Verbinden der Abschnitte des ersten und des zweiten Streifens, die sich über das Verbinden mit der zusätzlichen Unterlage hinaus erstrecken, miteinander;
wobei das funktionelle Verbinden in den Schritten r2), r4) und r6) durch Anwenden von Energie, vorzugsweise Wärmeenergie, bevorzugterweise Schallenergie und am bevorzugtesten Ultraschallenergie, ausgeführt wird, um Schmelzbindungen herzustellen.

9. Verfahren zur Herstellung eines absorbierenden Wegwerfartikels nach einem der Ansprüche 7 bis 8, ferner umfassend die Schritte des Aufbringens mindestens eines zusätzlichen Bahnmaterials, ausgewählt aus der Gruppe bestehend aus
t1) Flüssigkeitshandhabungsbahnmaterial, vorzugsweise ausgewählt aus der Gruppe bestehend aus
- Flüssigkeitaufnahmematerialien, die eine offene Porenstruktur aufweisen und vorzugsweise rückstellfähige Fasern, noch bevorzugterweise Polyesterfasern umfassen;
- Flüssigkeitsverteilungsmaterialien, die vorzugsweise Zellulosefasern oder schmelzgeblasene Fasern umfassen;
t2) einer zusätzlichen Oberlage;
t3) Seitenteilverlängerungen;
t4) Beinbändchen;
t5) Barrierebündchen,
wobei das zusätzliche Bahnmaterial / die zusätzlichen Bahnmaterialien durch Aufbringen von Energie, vorzugsweise Wärmeenergie, bevorzugterweise Schallenergie und am bevorzugtesten Ultraschallenergie funktionell verbunden wird / werden, um Schmelzverbindungen herzustellen.

## Revendications

1. Article absorbant jetable à porter sur le torse inférieur d'un porteur,
comprenant une zone d'entrejambe, a positionner dans la zone d'entrejambe d'un porteur pendant son utilisation et une région de taille frontale et arrière s'étendant vers l'avant et l'arrière vers la région de taille d'un porteur pendant l'utilisation,
l'article comprenant
une feuille de dessus perméable aux liquides;
une feuille de fond retenant les liquides; éventuellement une feuille de fond supplémentaire; au moins un premier et un second panneau latéral;
un élément de fermeture pour relier les régions de taille avant et arrière;
un noyau absorbant les liquides comprenant un matériau absorbant, de préférence superabsorbant, de préférence sous forme particulaire, comprenant en outre de préférence une première et une seconde nappes enveloppantes positionné entre ladite feuille de dessus et ladite feuille de fond;
**caractérisé en ce que** des liaisons par fusion relient de manière fonctionnelle
- ladite feuille de dessus, ladite feuille de fond et ledit noyau absorbant,
- et lesdits premier et second panneau latéral avec ladite feuille de dessus et ladite feuille de fond ;
et en plus **en ce que** lesdites liaisons par fusion sont essentiellement sans colle.

2. Article absorbant jetable selon la revendication 1, dans lequel
ledit noyau absorbant les liquides comprenant une première et une seconde nappes enveloppantes, reliées de manière fonctionnelle par des liaisons par fusion.

3. Article absorbant jetable selon une de la revendication 1 ou 2,
comprenant en outre un ou plusieurs matériau (s) en bande supplémentaire(s) qui est / sont reliés (s) de manière fonctionnelle à ladite feuille de dessus, ladite feuille de fond ou ladite noyau par des liaisons par fusion,
dans lequel
le ou les matériaux en bande(s) supplémentaire(s) est / sont choisis dans le groupe constitué par
- les bandes de manipulation des liquides, de préférence dans le groupe constitué par:
- des matériaux d'acquisition liquides présentant une structure à pores ouverts et comprenant de préférence des fibres résilientes, comprenant plus préférablement des fibres de polyester;
- et des matériaux de distribution de liquide comprenant de préférence des fibres cellulosiques ou des fibres soufflées à l'état fondu;
- une feuille de dessus supplémentaire;
- extensions de panneaux latéraux;
- cerceaux de jambe;
- revers de barrières.

4. Article absorbant selon l'une quelconque des revendications précédentes,
comprenant des liaisons de construction pour établir l'intégrité de l'article et éventuellement des liaisons d'application pour établir ou maintenir le positionnement de l'article sur un porteur,
dans lequel au moins les liaisons de construction sont uniquement des liaisons par fusion.

5. Article selon l'une quelconque des revendications précédentes,
l'article étant un article à porter sur le bas du torse d'un porteur, étant de préférence choisi dans le groupe constitué par
une couche ouverte, un article de style pantalon préalablement fermé et réouvrable ou un article de style pantalon fermé,
pour bébés, enfants en bas âge ou adultes.

6. Article selon l'une quelconque des revendications précédentes, plié intérieurement en quadruple.

7. Procédé de fabrication d'un article absorbant à porter sur le bas du torse de bébés, de enfants en bas âge ou d'adultes,
cet article étant sélectionné dans le groupe constitué par une couche ouverte, un article de style pantalon préalablement fermé et réouvrable ou un article de style pantalon fermé, l'article présentant une extension dans les directions x, y et z,
une ligne médiane longitudinale,
des régions de taille avant et arrière, chacune comprenant une première et une seconde régions en direction transversale et une région d'entrejambe,
une ligne médiane transversale dans la région d'entrejambe,
le procédé étant **caractérisé**
**en ce que** l'on fournit des pièces individuelles d'un noyau absorbant fabriqué selon les étapes
a) fournir
a1)
- matériau absorbant, de préférence superabsorbant, de préférence sous forme particulaire,
- une première et une seconde bande enveloppantes;
- éventuellement un matériau en bande supplémentaire pour une manipulation améliorée des liquides;
a2) un matériau de bande de feuille de fond présentant deux marges latérales s'étendant longitudinalement;
a3i) matériau de la feuille de dessus;
et matériau du panneau latéral;
b) préparer des morceaux individuels des noyaux absorbants en
b1) positionnant le matériau absorbant entre la première et la seconde bande enveloppantes;
b2) connecter fonctionnellement le premier et le seconde bande enveloppantes;
b3) séparer le matériau en bande relié pour former des morceaux individuels des noyaux absorbants;
comprenant en outre les étapes de
m) fournisser
m1) un matériau de bande de feuille de fond supplémentaire, présentant une ligne médiane longitudinale qui coïncide essentiellement avec la ligne médiane longitudinale de l'article, et des marges latérales s'étendant dans le sens longitudinal,
éventuellement composée de sous-bandes;
m3) éventuellement de matériau de bande d'extension de panneau latéral;
m4) éventuellement une bande de fermeture;
n) préparer un sous-ensemble de châssis à partir de la feuille de fond supplémentaire, éventuellement combiné avec le matériau en bande de panneau latéral et / ou le matériau de bande en extension de panneau latéral,
en appliquant des lignes de séparation à la feuille de fond supplémentaire, qui s'étendent principalement dans le MD et symétriquement à une ligne centrale s'étendant longitudinalement de la feuille de fond supplémentaire,
de sorte que, soit,
n1) des cerceaux de jambe sont formés par le matériau de feuille de fond supplémentaire, tandis que le matériau de panneau latéral et éventuellement le matériau d'extension de panneau latéral est / sont séparés du matériau de bande de panneau latéral ou d'extension de panneau latéral et fonctionnellement reliés à celle-ci,
la ligne de séparation étant positionnée au moins dans une partie de la région de l'entrejambe inférieure à 9 cm, de préférence inférieure à 3 cm, mais distante de plus de 0 cm des marges latérales s'étendant longitudinalement du matériau de feuille de fond supplémentaire,
et coupant une ligne de séparation supplémentaire, qui s'étend principalement dans le sens transversal du point d'intersection vers mais ne s'étendant pas dans la marge latérale du matériau de feuille de fond supplémentaire;
ou
n2) les panneaux latéraux et les cerceaux de jambe sont formés par le matériau de feuille de fond supplémentaire
et éventuellement les matériaux d'extension de panneau latéral sont séparés du matériau de bande d'extension de panneau latéral en morceaux et ajoutés et reliés fonctionnellement au panneau latéral de la feuille de fond supplémentaire,
la ligne de séparation est positionné au moins dans une partie de la région de l'entrejambe inférieure à 9 cm, de préférence inférieure à 3 cm mais éloignée de plus de 0 cm des bords latéraux de la feuille de fond supplémentaire s'étendant longitudinalement, et étant inférieure à 9 cm, de préférence inférieure à 3 cm, plus préférablement à moins de 1 cm de distance y inclus coïncidant avec la ligne médiane longitudinale dans les régions de taille avant et arrière, éventuellement en tant que ligne de séparation perf'n pop;
ou
n3) les extensions de panneau latéral, les panneaux latéraux et les cerceaux de jambe sont formés par le matériau de feuille de fond supplémentaire, les extensions de panneau latéral étant séparées du matériau de feuille de fond supplémentaire, formant ainsi des ensembles d'extension de panneau latéral,
la ligne de séparation étant positionnée au moins dans une partie de la région d'entrejambe inférieure à 9 cm, de préférence inférieure à 3 cm, mais éloignée de plus de 0 cm des bords latéraux de la feuille de fond s'étendant longitudinalement, et inférieure à 9 cm, de préférence inférieure à 3 cm, plus préférablement à moins de 1 cm de distance y inclus coïncidant avec la ligne médiane longitudinale dans les régions de taille avant et arrière,
éventuellement en tant que ligne de séparation perf'n pop;
o) combiner un sous-ensemble de châssis avec les morceaux individuels d'un noyau absorbant et la feuille de fond et la feuille de dessus,
repositionner ainsi les ensembles de extensions de panneau latéral, le cas échéant, vers la direction machine relative par rapport au matériau de feuille de fond supplémentaire restant, de sorte que
o1) la feuille de fond et la feuille de dessus enveloppent le noyau absorbant; et
o2) la feuille de fond supplémentaire est positionnée sur la feuille de fond du côté opposé du noyau absorbant; et
o3) les extensions de panneau latéral optionnelles sont positionnées entre la feuille de fond supplémentaire et la feuille de fond;
p) relier fonctionnellement
p1) la feuille de fond supplémentaire à la feuille de fond et / ou à la feuille de dessus;
p2) le cas échéant, le matériau du panneau latéral à la feuille de fond supplémentaire et / ou à la feuille de dessus;
p3) les panneaux latéraux faisant partie intégrale de la feuille de fond supplémentaire à la feuille de fond et / ou de la feuille de dessus à proximité des marges latérales longitudinales;
p4) le cas échéant, les extensions de panneau latéral aux panneaux latéraux;
p5) éventuellement le noyau absorbant de la feuille de dessus et / ou de la feuille de fond;
q) fournir un élément de fermeture pour l'article en
q1) ajouter et relier une bande de fermeture pour la fabrication d'une couche ouverte ou pour une couche pré-fermée et réouvrable;
ou
q2) positionner les marges latérales avant et arrière des parties de l'article dans un mode de butée ou de chevauchement pour former un article de style pantalon et relier les parties;
z) séparer un assemblage complet du composite continu de manière à former des articles individuels et éventuellement à poursuivre le traitement de tels articles,
dans lequel la connexion fonctionnelle se fait au moins aux étapes b2), c2), c3), p1), p2), p3) et p5), q) et p4) est exécuté en appliquant de l'énergie, de préférence de l'énergie thermique, plus préférablement de l'énergie sonique et plus préférablement de l'énergie ultrasonore pour établir des liaisons par fusion, qui sont essentiellement sans colle.

8. Procédé de fabrication d'un article absorbant jetable selon la revendication 7, dans lequel:
a3ii) l'étape de traitement a3) consistant à fournir la feuille de dessus comprend la fourniture d'une première et d'une seconde bande de matériau de bande de feuille de dessus,
chacune des bandes présentant une largeur dans la direction transversale supérieure à la moitié de la largeur du matériau de bande supplémentaire de la feuille de fond supplémentaire, le procédé comprenant en outre les étapes consistant à:
r1) positionner les première et seconde bandes de matériau de bande de la feuille de dessus de sorte que leur marge latérale orienté vers l'extérieur coïncide essentiellement avec des marges latérales orienté vers l'extérieur, respectivement, de la feuille de fond supplémentaire et telles que les marges des bandes opposées s'étendent au-delà de la ligne médiane s'étendant longitudinalement de l'article, de telle sorte que les bandes se chevauchent à proximité de la ligne médiane s'étendant longitudinalement;
r2) relier fonctionnellement les bandes de matériau de feuille de dessus avec le matériau de feuille de fond supplémentaire à proximité et le long des marges latérales s'étendant longitudinalement;
r3) sur-plier le bord latéral opposé et orienté vers l'extérieur de la bande de matériau de feuille de dessus au-delà de la connexion de la bande de matériau de feuille de dessus à la feuille de fond supplémentaire;
r4) relier de manière fonctionnelle les parties sur-pliées des bandes de matériau de feuille de dessus à la partie sous-jacente qui est déjà connectée à la feuille de fond supplémentaire;
r5) plier le sous-ensemble résultant le long de la ligne médiane longitudinale de telle sorte que les parties repliées de la première et de la deuxième bandes soient positionnées l'une vers l'autre et que les parties desdites bandes s'étendent au-delà de la feuille de fond supplémentaire;
r6) reliant fonctionnellement les parties de la première et de la deuxième bande, qui s'étendent au-delà de la connexion à la feuille de fond supplémentaire, l'une à l'autre;
dans lequel la connexion fonctionnelle dans les étapes r2), r4) et r6) est exécutée en appliquant de l'énergie, de préférence de l'énergie thermique, de manière davantage préférée de l'énergie sonique et de préférence de l'énergie ultrasonore pour établir des liaisons par fusion.

9. Procédé de fabrication d'un article absorbant jetable selon l'une quelconque des revendications 7 et 8, comprenant en outre les étapes consistant à appliquer au moins un matériau de bande supplémentaire choisi dans le groupe constitué par
t1) des bandes de traitement de liquide, de préférence choisi dans le groupe constitué par
- des matériaux d'acquisition de liquide présentant une structure à pores ouverts et comprenant de préférence des fibres résilientes, plus préférablement des fibres de polyester;
- matériaux de distribution de liquide comprenant de préférence des fibres cellulosiques ou des fibres soufflées à l'état fondu;
t2) une feuille de dessus supplémentaire;
t3) extensions de panneaux latéraux;
t4) cerceaux de jambe;
t5) des revers barrières,
dans lesquels le ou les matériaux en bande supplémentaire(s) sont reliés de manière fonctionnelle en appliquant de l'énergie, de préférence de l'énergie thermique, de manière davantage préférée de l'énergie sonique et de préférence de l'énergie ultrasonore pour établir des liaisons par fusion.
